(19) Europäisches Patentamt / European Patent Office / Office européen des brevets

(11) **EP 3 171 943 B1**

(12) **EUROPÄISCHE PATENTSCHRIFT**

(45) Veröffentlichungstag und Bekanntmachung des Hinweises auf die Patenterteilung:
**11.09.2019 Patentblatt 2019/37**

(21) Anmeldenummer: **15738115.3**

(22) Anmeldetag: **21.07.2015**

(51) Int Cl.:
**A61Q 15/00** (2006.01)   **A61Q 17/00** (2006.01)
**A61K 8/81** (2006.01)

(86) Internationale Anmeldenummer:
**PCT/EP2015/066594**

(87) Internationale Veröffentlichungsnummer:
**WO 2016/012418 (28.01.2016 Gazette 2016/04)**

(54) **DEODORANTZUBEREITUNGEN UMFASSEND POLYQUATERNIUM-16-POLYMERE**

DEODORANT PREPARATIONS COMPRISING POLYQUATERNIUM-16 POLYMERS

PRÉPARATIONS POUR DÉODORANTS, CONTENANT DES POLYMÈRES POLYQUATERNIUM-16

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **24.07.2014 DE 102014214463**

(43) Veröffentlichungstag der Anmeldung:
**31.05.2017 Patentblatt 2017/22**

(73) Patentinhaber: **Beiersdorf AG**
**20253 Hamburg (DE)**

(72) Erfinder:
- **TRAUPE, Bernd**
  **24568 Kaltenkirchen (DE)**
- **WEETS, Gudrun**
  **22527 Hamburg (DE)**
- **GROTH, Inken**
  **20257 Hamburg (DE)**
- **FIRYN, Andreas**
  **21493 Schwarzenbek (DE)**
- **KLAWITER, Helen**
  **20255 Hamburg (DE)**
- **KLAUCK, Robert**
  **21465 Reinbek (DE)**
- **PRIEBE, Laura**
  **22049 Hamburg (DE)**
- **BRAREN, Sandra**
  **20251 Hamburg (DE)**
- **FOELSTER, Heike**
  **22149 Hamburg (DE)**
- **KANINCK, Stefanie**
  **22359 Hamburg (DE)**
- **LIEBICH, Anne**
  **22047 Hamburg (DE)**

(56) Entgegenhaltungen:
| | |
|---|---|
| EP-A2- 0 200 548 | WO-A1-94/01077 |
| WO-A1-2005/087188 | WO-A1-2010/070140 |
| DE-A1- 19 710 155 | DE-A1-102010 007 958 |
| JP-A- 2012 177 901 | JP-A- 2014 101 356 |
| US-A- 4 478 821 | US-A1- 2007 191 548 |
| US-A1- 2007 197 418 | US-A1- 2009 061 004 |

- "International Cosmetic Ingredient Dictionary and Handbook", 2012, Personal Care Products Council, XP002743795, Seite 2565,2568, Eintrag "POLYQUATERNIUM-16"
- Anonymous: "Deodorant", WikiPedia , 4. Juli 2015 (2015-07-04), Seiten 1-4, XP055208564, Gefunden im Internet: URL:https://de.wikipedia.org/wiki/Deodoran t [gefunden am 2015-08-19]
- WENDEL V: "Formulation guide - Luvigel Star - Skin Care Rheological Modifier with Associative Thickening Mechanism", 20090401 , 1 April 2009 (2009-04-01), pages 1-36, XP002612052, Retrieved from the Internet: URL:http://www.univarusa.com/vwr-inc/tools .n sf/0/8D98A1C2811DFAD88825777E0070947C/$f ile/090713_%20formulation%20guide_Luvigel% 20Star_customer%20(incl%20visco_pH).pdf [retrieved on 2010-11-15]

**Beschreibung**

Die Erfindung ist die Verwendung von Polyquaternium-16 Polymeren gemäß Anspruch 1 als Deodorantwirkstoff.

Antitranspirantien (AT) oder Desodorantien (Deo) dienen dazu, Körpergeruch zu beseitigen oder zu verhindern, der entsteht, wenn der an sich geruchlose frische Schweiß durch Mikroorganismen zersetzt wird. Den üblichen kosmetischen Desodorantien liegen unterschiedliche Wirkprinzipien zugrunde.

Im allgemeinen Sprachgebrauch erfolgt nicht immer eine klare Trennung der Begriffe "Deodorant/Desodorant" und "Antitranspirant". Vielmehr werden - insbesondere auch im deutschsprachigen Raum - Produkte zur Anwendung im Achselbereich pauschal als Desodorantien bzw. "Deos" bezeichnet. Dies geschieht unbeachtlich der Frage, ob auch eine antitranspirante Wirkung vorliegt.

Antitranspirantien (AT) sind schweißverhütende Mittel, die, im Gegensatz zu den Desodorantien, die Absonderung von Schweiß überhaupt verhindern sollen. Desodorantien verhindern im Gegensatz dazu im Allgemeinen eine mikrobielle Zersetzung von bereits gebildetem Schweiß.

Im Gegensatz zu den Antitranspirantien bewirken reine Desodorantien keine aktive Beeinflussung der Schweißsekretion, sondern lediglich die Steuerung bzw. Beeinflussung des Körper- bzw. Achselgeruches (Geruchsverbesserungsmittel). Schweißgeruch besteht zu einem Großteil aus verzweigtkettigen Fettsäuren, die durch bakterielle Enzyme aus geruchlosem Schweiß freigesetzt werden. Klassische Deo-Wirkstoffe wirken dem entgegen, indem sie das Wachstum von Bakterien reduzieren.

Entscheidend für eine desodorierende Wirkung ist es dabei, wenn desodoriende Wirkstoffe vornehmlich gegen die Bakterien Staphylococcus epidermidis und Corynebacterium jeikeium wirken.

Polyquaternium Polymere sind nach der INCI-Nomenklatur Bezeichnungen für komplexe quartäre Ammonium-Polymerverbindungen. Die verschiedenen Vertreter dieser Gruppe werden durch Ziffern unterschieden. Zum Beispiel Polyquaternium-1, Polyquaternium-16 (PQ-16), Polyquaternium-42, usw. Als quartäre Ammoniumverbindungen besitzen Polyquaternium-Polymere quartäre Stickstoffatome, d. h. alle vier Wasserstoffatome des Ammonium-Ions sind durch organische Reste ersetzt. Die Verbindungen sind somit kationisch, besitzen also eine positive Ladung.

In der WO 2013052454 A1 ist eine Übersicht bekannter Polyquaternium Polymere und deren Handelsnamen aufgeführt.

Vertreter dieser nach INCI benannten Gruppe sind u. a. wegen ihrer filmbildenden und antistatischen Eigenschaften in vielen Körperpflegeprodukten enthalten, z. B. in Shampoos.

. Polyquaternium-16 Polymere sind 3-Methyl-1-Vinylimidazolium Chloride-1-Vinyl-2-Pyrroli-dinone Chloride, wie sie unter den Handelsnamen Luviquat® angeboten werden, wie in nachfolgender Tabelle 1 dargestellt:

| Handelsname | Feststoffgehalt % | Molmasse | Verhältnis VP/QVI | Ladungsdichte (meq/g) |
|---|---|---|---|---|
| Luviquat Excellence | 38-42 | 40,000 | 5/95 | 6,1 |
| Luviquat FC 550 | 38-42 | 80,000 | 50/50 | 2,0 |
| Luviquat FC 370 | 38-42 | 100,000 | 70/30 | 3,3 |
| Luviquat Style | 19-21 | 400,000 | 55/45 | 3,0 |

In der EP 1504744 B1 werden Polyquaternium-16 Polymere im Bereich der Haarpflege, insbesondere in Haarsprays, angewendet.

In der US 20070184016 A1 und WO 2007095008 A2 werden Polyquaternium-16 Polymere in Desinfektionsmitteln beschrieben, wie auch alkoholische Lösungen. Diese umfassen jedoch einen Alkoholgehalt von mindestens 50%. Aufgrund der bekannten hohen kationischen Ladungsdichte der Polymere ist zur homogenen Stabilisierung im Produkt der Einsatz einer großen Menge an Ethanol notwendig.

In der EP 1991654 A1 werden Polyquaternium-16 Polymere, z.B. unter dem Namen Luviquat FC 905 erhältlich, in Seifenstückmasse beschrieben.

DE 102010007958 A1 offenbart Wirkstoffkombinationen aus einem oder mehreren Acylarginaten und einer oder mehreren quaternären Ammoniumverbindungen. In Absatz [0053] wird in einer Auflistung Polyquaternium-16 genannt. Jedoch wird kein spezifischer Hinweis auf die Polyquaternium-16 Polymere gemäß Anspruch 1 gegeben.

Ferner offenbaren die Dokumente JP 2014 101356 A und WO 9401077A1 verschiedene kosmetische Rezepturen enthaltend Polyquaternium-16. Jedoch wird auch hier kein spezifischer Hinweis auf die vorliegende Erfindung gegeben.

Die EP 200548 A2 beschreibt Deodorantzubereitungen umfassend antimikrobielle Wirkstoffe, wie Triclosan oder Benzalkoniumchlorid. Die Zubereitungen umfassen des Weiteren kationische Polymere, insbesondere Copolymere der Dimethyldiallyammoniumchloride (DMDAAC), wie Merquat S® oder Merquat 550® (PQ-7).

Diese kationischen Polymere verbessern die antimikrobielle Wirksamkeit der antimikrobiellen Wirkstoffe aufgrund ihrer Substantivität und Filmbildungseigenschaften. D.h. sie sorgen dafür, dass die antimikrobiellen Wirkstoffe länger am Wirkort verbleiben können und somit erst eine verbesserte Wirksamkeit zeigen können. Ein Hinweis darauf, dass Polyquaternium Polymere per se eine antimikrobielle Wirksamkeit und ggf. eine desodorierende Wirkung aufweisen, ist nicht gegeben.

Schweißhemmung aufgrund der Bildung okklusiver Filme wird in der WO 1995/24105 A1 und Schweißhemmung aufgrund adstringierender und flüssigkeitsabsörbierender Wirkungen wird in der US 4743440 und WO 2003/030853 A2 beschrieben.

In der WO 2009091794 A1 werden Zubereitungen beschrieben, die neben antitranspirant wirksamen Mitteln kationische Polyquaternium Polymere, PQ-6, PQ-7, PQ-10, PQ-11, PQ-22 und insbesondere PQ-28, umfassen.

In der EP 1761241 A1 werden antitranspirant wirksame Stoffe auf Basis von Diallydimethylammoniumchloriden (DADMAC) und Diallyethylammoniumchloriden (DADEAC) beschrieben, insbesondere PQ-6 (Merquat® 100, Flocare® C106, Floquat FL45®), Polyquaternium-33 (Floerger FO 4190 VHM®,4550 BPM®), Polyquaternium-5 (Merquat 5®, Reten®) und Polyquaternium-7 (Merquat 550 L®, Salcare Super 7®, Flocare C107®). PQ-16 Polymere sind hierin nicht beschrieben.

Ein Hinweis auf eine desodorierende Wirksamkeit dieser Stoffe ist damit nicht gegeben, da es einen eklatanten Unterschied im Wirkmechanismus zwischen Schweißhemmung (antitranspirant wirksam) und Geruchsverbesserungsmittel (desodorierend) gibt.

In der JP 2014101356 wird die Kombination von Polyquaternium-10 mit antimikrobiellen Stoffen, wie Benzalkoniumchlorid oder Isoropylmethylphenol, als Waschmittel beschrieben, die eine Körpergeruchsverbesserung, insbesondere für ältere Menschen, hervorrufen soll.

In den Vergleichstests wird eine Zubereitung mit Polyquaternium-10 und ohne antimikrobiellem Mittel als nicht geruchsverbessernd angesehen.

In der Zeitschrift für Fett-, Öl-, Kosmetik-, Pharma- und Waschmittelindustrie, 111. Jahrgang, Nr. 4, vom 6. März 1985 werden verschiedene polyquaternäre Polymere mit abgestufter Kationenaktivität vorgestellt, die unter der Handelsbezeichnung Luviquat 905, 550 und 370 der BASF erhältlich sind. Die Polymere unterscheiden sich durch die Kombination mit Vinylpyrrolidon (VP) und quaternisiertem Vinylimidazol (VI, QVI). Ihr Einsatz ist für Haarzubereitungen beschrieben.

Probleme ergeben sich beim Einsatz von Polyquaternium Polymeren, insbesondere PQ-16, aufgrund ihrer hohen kationischen Ladungsdichte, wodurch es in den Zubereitungen zu Ausfällungen, Agglomerationen und Phasenbildungen kommen kann.

Wünschenswert ist es daher homogene, stabile Formulierungen, die Polyquaternium-16 Polymere umfassen und die auch unter thermischer Belastung ihr Erscheinungsbild nicht verändern, zur Verfügung zu stellen.

Wünschenswert ist es darüber hinaus O/W-Emulsionen, unterscheidbar in Mikro-und Makroemulsionen, umfassend PQ-16 Polymere, bereit zu stellen.

Im Stand der Technik sind kosmetische Zubereitungen, beispielsweise als Deodorantien oder Antitranspirantien, beschrieben, die ein oder mehrere Polyquaternium Polymere umfassen. Damit ist jedoch noch nicht offenbart, dass die Polyquaternium Polymere per se als Deodorantwirkstoff agieren.

Die Anwendungsformen und Arten von Antitranspirantien und Deodorantien unterliegen häufig dem Zeitgeschmack. In einer mehr und mehr hektischen Umgebung sollte das Pflegen, wie das Desodorieren, möglichst wenig Zeit in Anspruch nehmen. Des Weiteren wird zunehmend eine "Zwischendurch-Anwendung" von Deodorant- oder Antitranspirantmittel gewünscht.

Wünschenswert ist es kosmetische Zubereitungen als Desodorant bereit zu stellen, die ohne die geschilderten Nachteile des Standes der Technik angewendet werden können. Insbesondere ist es wünschenswert kosmetische Desodorantien bereit zu stellen, die auch auf der Kleidung angewendet werden können und dennoch kosmetisch wirksam sind.

Die Erfindung ist die Verwendung von Polyquaternium-16 Polymeren gemäß Anspruch 1 als Deodorantwirkstoff.

Dazu werden die Polyquaternium-16 Polymere vorzugsweise in kosmetischen oder dermatolgischen Zubereitungen bereitgestellt. In dieser Hinsicht ist es erfindungsgemäß, dass diese Zubereitungen, so wie auch die im Weiteren beschriebenen, in den beschriebenen Verwendungen zum Einsatz kommen.

Polyquaternium Polymere sind als Filmbildner insbesondere in Haarzubereitungen bekannt.

Zu erwarten ist daher, dass kosmetische Zubereitungen umfassend Polyquaternium Polymere sich auf der Haut und Haaren fest anlagern bzw. aufziehen.

Erstaunlicherweise lassen sich die Zubereitungen umfassend ein oder mehrere Polyquaternium-16 Polymere, insbesondere als Mikroemulsion formuliert besonders leicht von der Haut wieder entfernen. Sie lassen sich erstaunlicherweise auch leicht wieder abwaschen.

Aus der Färbetechnologie von Textilien ist bekannt, dass kationische Farbstoffe auf natürlichen Fasern nur mäßig waschechte Farben bilden, da sie nur mäßig gut auf den Fasern binden.

Erstaunlicherweise binden jedoch Polyquaternium-16 Polymere, obwohl sie kationische Ladungen tragen, sehr gut auf Textilfasern. In Untersuchungen zur desodorierenden Wirksamkeit konnte gezeigt werden, dass Polyquaternium Polymere aufgebracht auf Textilien zu einer signifikanten Geruchsverbesserung führen.

Was dazu führt, dass erfindungsgemäße Zubereitungen auch auf Textilien aufziehen können und auch dort den Schlechtgeruch bekämpfen können.

Die Verwendung von ein oder mehreren Polyquaternium-16 Polymeren zur Desodorierung auf oder durch die Kleidung ist damit möglich.

Mittels der Zubereitung kann das Kosmetikum auf die Haut appliziert werden, wobei vorteilhaft dies direkt durch die Kleidung erfolgen kann.

Als geeignete Kleidung sind in diesem Zusammenhang selbstverständlich diejenigen Kleidungsformen, die aufgrund ihrer Materialien oder deren Herstellung keinerlei Substanz aufnehmen oder hindurch lassen, ausgenommen.

Die Polyquaternium-16 Polymere zeigen eine desodorierende Wirksamkeit. Im Gegensatz zu den Antitranspirantien bewirken die Desodorantien keine aktive Beeinflussung der Schweißsekretion, sondern lediglich die Steuerung bzw. Beeinflussung des Körper- bzw. Achselgeruches (Geruchsverbesserungsmittel). In diesem Sinne werden die erfindungsgemäßen Polyquaternium-16 Polymere (PQ-16) gemäß Anspruch 1 als Deodorantien verwendet.

Die Zubereitungen können dabei ein oder mehrere weiteren desodorierend und/oder antitranspirant wirkende Stoffe umfassen. Bevorzugt ist es aber, dass die erfindungsgemäß verwendeten Zubereitungen insbesondere keine weiteren antitranspirant wirkende Stoffe umfassen, insbesondere keine Aluminiumsalze.

Des Weiteren sind die Zubereitungen bevorzugt frei von organischen Verbindungen mit anionischer Ladung. Die klassischen Gegenanionen, wie Chloridionen oder Bromidionen, sind nicht als organische Verbindungen mit negativer Ladung zu verstehen. Bevorzugt wird auf die Anwesenheit von lipophilen organischen Verbindungen, insbesondere Polymeren mit anionischer Ladung verzichtet, da es ansonsten zu Agglomeration, Ausfall und Instabilitäten kommen kann. Als Verbindungen mit anionischer Ladung werden Verbindungen verstanden, die eine negativ geladene funktionelle Gruppe besitzen.

Des Weiteren ist der Zusatz an weiteren antimikrobiellen Stoffen nicht zwingend erforderlich.

Hinsichtlich der Lagerstabilität können selbstverständlich Konservierungsverbesserer zugesetzt werden, wie beispielsweise Octopirox oder Phenoxyethanol.

Da die erfindungsgemäßen Polyquaternium-16 Polymere desodorierende Wirkungen aufweisen, kann auch auf den Zusatz anderer Deodorantwirkstoffe bevorzugt verzichtet werden.

Obwohl Parfumstoffe auch als Geruchsverbesserer gelten, sind Parfumstoffe hierbei explizit ausgenommen und die erfindungsgemäßen Zubereitungen können Parfum mit umfassen.

Aufgrund der Stabilitätsproblematik von Zubereitungen umfassend Polyquaternium Polymeren mit hoher kationischer Ladungsdichte umfasst die vorliegende Erfindung einen weiteren Aspekt der Stabilisierung PQ-16 haltiger Zubereitungen.

Die Stabilisierung konnte erfindungsgemäß wie nachstehend ausgeführt gelöst werden.

Die Erfindung ist daher auch die Verwendung einer kosmetischen oder dermatologischen Zubereitung umfassend

a.) ein oder mehrere Polyquaternium-16 Polymere gemäß Anspruch 1 und ein oder mehrere Stoffe gewählt aus den Gruppen b.) nichtionischen Emulgatoren, c.) kationischen Emulgatoren und d.) Rheologiemodifizierer.

Die Stoffgruppe b.) umfasst ein oder mehrere nichtionische Emulgatoren. Der Gesamt HLB Wert der nichtionischen Emulgatoren, eines einziges nichtionischen Emulgators oder einer Mischung zweier oder mehrerer nichtionischer Emulgatoren, liegt dabei bevorzugt im Bereich von größer 9 bis zu 17. Ist die Zubereitung auf Basis einer Makroemulsion formuliert liegt der Gesamt HLB-Wert der Emulgatormischung idealerweise zwischen 3 bis 14, insbesondere im Bereich 4 bis 12, bevorzugt im Bereich 7 bis 10.

D.h. es kann einerseits ein einziger nichtionischer Emulgator mit einem HLB Wert größer 9 und bis zu 16 bzw. 3 bis 14 gewählt werden (z.B. Laureth-4 - HLB 9,7) oder mehrere nichtionische Emulgatoren mit unterschiedlichen HLB - Werten, wobei der HLB - Wert der Mischung dann in dem erfindungsgemäßen Bereich liegen sollte (z.B. Glyceryl Isostearate HLB 3,5 und Isoceteth-20 (HLB 15.7)). Dabei spielt auch deren jeweiliger Anteil und das Verhältnis zueinander eine erfindungsgemäße Rolle, wie nachfolgend ausgeführt wird.

Die Stoffgruppe c.) umfasst ein oder mehrere kationische Emulgatoren und die Stoffgruppe d.) ein oder mehrere Rheologiemodifizierer.

Rheologiemodifizierer (d.) sind Stoffe, die die Viskosität der sie enthaltenen Zubereitung beeinflussen, insbesondere erhöhen.

Die Zubereitungen sind bevorzugt frei von organischen Verbindungen mit anionischer Ladung.

Frei von bedeutet dabei, dass der Anteil an diesen Stoffen auf die verzichtet werden kann, wie anionische Stoffe, antitranspirant wirksame Stoffe oder zusätzliche Deodorantien, weniger als 0,1 Gew.%, bezogen auf die Gesamtmasse der Zubereitung, beträgt. Dadurch wird gewährleistet, dass Einschleppungen oder Verunreinigungen mit diesen Stoffen nicht als erfindungsgemäß "frei von" mitumfasst wird.

Als erfindungsgemäß haben sich Polyquaternium-16 Verbindungen (PQ-16) herausgestellt, die durch folgende Parameter gekennzeichnet sind:

- Monomerverhältnis VP/QVI (Gew.%): 4 - 8 / 92 - 96, insbesondere 7/93 bis 5/95, insbesondere im Bereich von 5 bis 95% (VP/QVI),
- Ladungsdichte (meq/g): 5 bis 6,5, bevorzugt zwischen 5,8 und 6,2
- Molmasse (Da): 10.000 - 1.000.000, bevorzugt 20.000 - 80.000.

Die Handelsbezeichnungen Luviquat® umfassen erfindungsgemäß bevorzugten PQ-16 Polymere, wie in Tabelle 1 angegeben. Der Aktivgehalt an PQ-16 Polymeren (Feststoffanteil) liegt dabei im Bereich von 19 bis 42 Gew.%. Der Rest besteht aus Wasser.

Der Anteil an einem oder mehreren Polyquaternium-16 Polymeren in kosmetischen oder dermatologischen Zubereitungen beträgt vorteilhaft insgesamt bis zu 10 Gew.%, bevorzugt bis zu 2 Gew.% und minimal 0,01 Gew.%, jeweils bezogen auf die Gesamtmasse der Zubereitung.

Der Anteil an einem oder mehreren nichtionischen Emulgatoren (b.), kationischen Emulgatoren (c.) und/oder Rheologiemodifizierer (d.) liegt jeweils vorzugsweise im Bereich bis zu 10 Gew.%, insbesondere im Bereich von 0,1 bis 8 Gew.%, bezogen auf die Gesamtmasse der Zubereitung.

Bevorzugt sind die Zubereitungen als Mikroemulsion, wässrig-alkoholischen Formulierungen oder Makroemulsionen formuliert.

Erfindungsgemäße Polyquaternium-16 Polymere zeigen eine gute Deowirksamkeit, wie in nachfolgenden Untersuchungen gezeigt wird. Es ist damit generell eine Deowirksamkeit von Polyquaternium-16 Polymeren erstmalig gezeigt worden.

Der Einsatz von Polyquaternium-16 Polymeren als Deodorantwirkstoff in kosmetischen Deodorantzubereitungen ist damit möglich und erfindungsgemäß.

Aufgrund der relativ hohen kationischen Ladungsdichte der PQ-16 Verbindungen ist eine Einarbeitung in kosmetischen Formulierungen problematisch, da es zu Ausfällungen und/oder Agglomerationen von Emulgator- bzw. Verdickersystemen der Zubereitungen oder es sogar zur Inaktivierung der Wirkstoffe kommen kann.

Besonders bei opaken Formulierungen, beispielsweise opaken Makroemulsionen, kann die Stabilität durch die Anwesenheit von Polyquaternium Polymeren stark negativ beeinflusst werden.

Es erfolgt eine Stabilisierung kosmetischer Zubereitungen umfassend Polyquaternium-16 Verbindungen, insbesondere in Mikroemulsionen, dabei vornehmlich Mikroemulsionsgele, und bevorzugt wässrig-alkoholische Formulierungen und Makroemulsionen, indem diesen Zubereitungen die Emulgatoren (nichtionische Emulgatoren b.) und/oder kationische Emulgatoren c.)) und/oder Rheologiemodifizierer (d.), welche keine negative Ladung tragen, zugesetzt werden. D.h. es werden kationische und/oder nichtionische Emulgatoren und/oder kationische und/oder Rheologiemodifizierer, bevorzugt nichtionische Modifizierer, bevorzugt zur Stabilisierung eingesetzt.

Erfindungsgemäß bevorzugt ist eine Makroemulsion, die Emulgatoren und/oder Rheologiemodifizierer, welche keine negative Ladung tragen, zugesetzt werden. D.h. es werden kationische und/oder nichtionische Emulgatoren bzw. kationische und/oder nichtionische Rheologiemodifizierer zur Stabilisierung der Makroemulsion eingesetzt.

Die Emulgatoren werden aus der Gruppe mit einem HLB-Wert im Bereich von 1 bis 20 gewählt.

Bevorzugt umfassen die Zubereitungen zwei nichtionische Emulgatoren (b.).

Insbesondere werden nichtionische Emulgatoren gewählt, so dass der Gesamt HLB Wert der Emulgatoren im Bereich von 3 bis 17, bevorzugt 4 bis 16, insbesondere 9 bis 16, insbesondere 10 bis 15, insbesondere im Bereich von 10 bis 14 liegt.

In einer Makroemulsion liegt der Gesamt HLB-Wert der Emulgatoren bevorzugt im Bereich von 4 bis 12, idealerweise im Bereich 7 bis 10.

Emulgatoren bewirken, dass zwei nicht miteinander mischbare Flüssigkeiten (zum Beispiel Öl in Wasser) sich zu einer Emulsion vermengen können. Aufgrund des amphiphilen Charakters dringen sie mit ihrem fettlöslichen Teil in das Öl ein. Durch den hydrophilen Teil kann das nun durch Rühren entstandene Öltröpfchen in der wässrigen Umgebung dispergiert werden. Emulgatoren haben primär keinen waschaktiven, tensidischen Charakter.

Emulgatoren setzen die Grenzflächenspannung zwischen den beiden Phasen herab und erreichen neben der Verringerung der Grenzflächenarbeit auch eine Stabilisierung der gebildeten Emulsion. Sie stabilisieren die gebildete Emulsion durch Grenzflächenfilme sowie durch Ausbildung sterischer oder elektrischer Barrieren, wodurch das Zusammenfließen (Koaleszenz) der emulgierten Teilchen verhindert wird.

Damit Verbindungen als Emulgatoren wirksam sein können, müssen sie eine bestimmte Molekülstruktur aufweisen. Strukturelles Kennzeichen solcher Verbindungen ist ihr amphiphiler Molekülaufbau. Das Molekül einer solchen Verbindung besitzt wenigstens eine Gruppe mit Affinität zu Substanzen starker Polarität (polare Gruppe) und wenigstens eine Gruppe mit Affinität zu unpolaren Substanzen (apolare Gruppe).

Es wird dabei unterschieden zwischen nicht-ionischen, anionischen und kationischen Emulgatoren. Ein Kennzeichen für die Hydrophilie eines gegebenen Emulgators ist dessen HLB-Wert, der sich nach folgender Formel ergibt: HLB = 20 x (1- $M_{lipophil}$/M), wobei $M_{lipophil}$ für die Molmasse des lipophilen Anteils im Emulgator und M für die Molmasse des gesamten Emulgators steht.

Im Allgemeinen gelten Emulgatoren mit einem HLB-Wert bis ca. 8 als W/O-Emulgatoren. O/W-Emulgatoren hingegen weisen HLB-Werte von größer 8 bis 15 auf. Substanzen mit HLB-Werten größer 15 werden häufig als Lösungsvermittler, die hier erfindungsgemäß auch als Emulgatoren zu wählen sind, bezeichnet.

Beispiele erfindungsgemäß bevorzugter nichtionischer Emulgatoren (b.) sind Glycol Distearate (HLB 1.0), Sorbitan Trioleate (HLB 1.8), Propylene Glycol Isostearate (HLB 2.5), Glycol Stearate (HLB 2.9), Glyceryl Isostearate (HLB 3,5), Sorbitan Sesquioleate (HLB 3.7), Glyceryl Stearate (HLB 3.8), Lecithin (HLB 4), Sorbitan Oleate (HLB 4.3), Sorbitan Monostearate NF (HLB 4.7), Sorbitan Stearate (HLB 4.7), Sorbitan Isostearate (HLB 4.7), Steareth-2 (HLB 4.9), Oleth-2 (HLB 4.9), Glyceryl Laurate (HLB 5.2), Ceteth-2 (HLB 5.3), PEG-30 Dipolyhydroxystearate (HLB 5.5), Glyceryl Stearate SE (HLB 5.8), Sorbitan Stearate (and) Sucrose Cocoate (HLB 6), PEG-4 Dilaurate (HLB 6), PEG-8 Dioleate (HLB 8), Sorbitan Laurate (HLB 8.6), PEG-40 Sorbitan Peroleate (HLB 9), Laureth-4 (HLB 9.7), PEG-7 Glyceryl Cocoate (HLB 10), PEG-20 Almond Glycerides (HLB 10), PEG-25 Hydrogenated Castor Oil (HLB 10.8), Stearamide MEA (HLB 11), Glyceryl Stearate (and) PEG-100 Stearate (HLB 11), Polysorbate 85 (HLB 11), PEG-7 Olivate (HLB 11), Cetearyl Glucoside (HLB 11), PEG-8 Oleate (HLB 11.6), Polyglyceryl-3 Methyglucose Distearate (HLB 12), Oleth-10 (HLB 12.4), Oleth-10 / Polyoxyl 10 Oleyl Ether NF (HLB 12.4), Ceteth-10 (HLB 12.9), PEG-8 Laurate (HLB 13), Ceteareth-12 (HLB 13,5), Cocamide MEA (HLB 13.5), PEG-30 Glyceryl Laurate (HLB 14), Polysorbate 60 NF (HLB 14.9), Polysorbate 60 (HLB 14.9), Polysorbate 80 (HLB 15), PEG-40 Hydrogenated Castor Oil (HLB 15), Isosteareth-20 (HLB 15), PEG-60 Almond Glycerides (HLB 15), Polysorbate 80 NF (HLB 15), PEG-20 Methyl Glucose Sesquistearate (HLB 15), Ceteareth-20 (HLB 15.2), Oleth-20 (HLB 15.3), Steareth-20 (HLB 15.3), Steareth-21 (HLB 15.5),Ceteth-20 (HLB 15.7), Isoceteth-20 (HLB 15.7), Polysorbate 20 (HLB 16.7), Polysorbate 20 NF (HLB 16.7), Laureth-23 (HLB 16.9), PEG-100 Stearate (HLB 18.8), Steareth-100 (HLB 18.8), PEG-80 Sorbitan Laurate (HLB 19.1), PEG-150Laurate(HLB 19.3).

Bevorzugt werden dabei Glyceryl Isostearate, Glyceryl Stearate, Steareth-2, Ceteareth-20, Steareth-21, PEG-40 Hydrogenated Castor Oil, PG-10 Stearate, Isoceteth-20, Isosteareth-20 und Ceteareth-12.

Als Lösungsvermittler bekannt aber als erfindungsgemäße Emulgatoren sind des Weiteren bevorzugt zu wählen PEG-40 Hydrogenated Castor Oil, Polysorbate 80, Laureth-23, PEG-150Laurate und PEG-30 Glyceryl Laurate.

Neben bzw. anstelle nichtionischer Emulgatoren b.) sind auch kationische Emulgatoren c.) geeignet um stabile Formulierungen mit Polyquaternium-16 Polymeren zu erstellen. Bevorzugte geeignete kationische Emulgatoren c.) sind zu wählen aus der Gruppe Cetrimonium Chloride, Palmitamidopropyltrimonium Chloride, Quaternium-87, Behentrimonium Chloride, Distearoylethyl Dimonium Chloride, Distearyldimonium Chloride, Stearamidopropyl Dimethylamin und/oder Behentrimonium Methosulfat.

Beispiele erfindungsgemäßer Rheologiemodifizierer d.) sind natürliche organische Polymere und ihre Derivate, Gummi arabicum, Karaya, Tragant, Johannisbrotkernmehl, Guar, Pektin, Agar agar, Carrageen, Alginate, Xanthan, Stärke und Stärkederivate, Cellulose und Cellulosederivate, wie Microcristalline Cellulose, Methylcellulose, Cellulose Gum, Hydroxyethylcellulose, Hydroxylpropylcellulose, Methylhydroxypropylcellulose, desweiteren anorganische Gelbildner, wie Silikate, z.B. Bentonite, Hectorite, kolloidale Kieselsäure; darüber hinaus synthetische Verdicker z.B. Poylvinylalkohol und ebenso ethoxylierte Verbindungen wie Poloxamer, PEG-150 Distearate, PEG-120 Methyl Glucose Dioleate, PEG-9 Dilaurate und Fettalkohole, wie Stearylalkolhol, Cetylalkohol und Cetearylalkohol.

Werden beispielsweise negativ geladene Stoffe, wie anionische Emulgatoren eingesetzt, so wird keine Stabilisierung, sondern Agglomerationen und Phasenbildungen beobachtet.

Der alleinige Verzicht auf anionische Emulgatoren, Tenside, Solubilisatoren und Rheologiemodifizierer führt dabei nicht automatisch zu den erfindungsgemäßen stabilen Formulierungen. Vielmehr wurde gefunden, dass es bevorzugt ist eine definierte Polarität des Emulgatorsystems zu wählen (nichtionisch und HLB im bevorzugten Bereich, z.B. 7 bis 16, und/oder kationisch) neben der Abwesenheit von anionischen Ladungen um die Zubereitungen mit Polyquaternium-16 zu stabilisieren.

Es wird daher bevorzugt auf die Anwesenheit organischer Verbindungen mit anionischer Ladung verzichtet und die Emulgatoren im erfindungsgemäßen HLB-Wertebereich gewählt.

Erstaunlicherweise konnte eine nochmals verbesserte Stabilität von Polyquaternium-16 Polymeren enthaltene Zubereitungen erreicht werden, wenn erfindungsgemäß der Einsatz von nichtionischen Emulgatoren oder eines Solubilisierers (Lösevermittlers) mit einem HLB-Wert von mindestens 8, bevorzugt mindestens 9, optional in Kombination mit Emulgatoren mit einem HLB-Wert kleiner 8, insbesondere kleiner 9, erfolgt, wobei auch hier der Gesamt HLB Wert aller Emulgatoren und Lösevermittler im bevorzugten Bereich liegt.

Erfindungsgemäß bevorzugt liegt bei einer Kombination von Emulgatoren der lipophilere Emulgator im Verhältnis von 7:1 bis 1:7 zum hydrophileren Emulgator vor, bevorzugt im Verhältnis 6:1 bis 1:6, bevorzugt im Verhältnis 5:1 bis 1:5, bevorzugt im Verhältnis 4:1 bis 1:4.

Erwünscht und bevorzugt ist ein so resultierender HLB-Wert des gesamten Emulgatorsystems im Bereich von 3 - 15 und bevorzugt von 4 bis 12.

Als Emulgatoren mit einem HLB-Wert kleiner 9 werden beispielsweise Glyceryl Isostearate, Glyceryl Stearate und Steareth-2 bevorzugt.

Als hydrophilere Emulgatoren mit einem HLB-Wert von mindestens 9 werden bevorzugt Ceteareth-20, Steareth-21, PEG-40 Hydrogenated Castor Oil, PG-10 Stearate, Isoceteth-20, Isosteareth-20 und Ceteareth-12.

Bei transparenten Mikroemulsionen und opaken Makroemulsion zeigt sich diese optimale Kombination von Emulgator und Co-Emulgator stabilisierend, optional durch additiven Einsatz eines nichtionischen/kationischen Rheologiemodifizierers. D.h. bei einer transparenten Mikro- oder Makroemulsion sind zwei erfindungsgemäße nichtionische Emulgatoren und optional ein Rheologiemodifizierer zuzusetzen.

Der Zusatz an PQ-16 in transluzenten PIT-Formulierungen konnte erfindungsgemäß ebenfalls gelingen, da diese, wie bei der Mikro- und Makroemulsion, durch Zusatz an nichtionischen Emulgatoren stabilisiert werden können.

Bei wässrig-alkoholischen Formulierungen zeigt sich für die Stabilisierung der Zusatz eines Lösungsvermittlers erfolgsversprechend. Insbesondere konnte dabei der Anteil an C1-C6-Alkoholen, insbesondere ein Ethanolgehalt, reduziert werden. Ein Ethanolgehalt von 30% bis zu 45% ist hierbei ausreichend, obwohl im Stand der Technik nur Zubereitungen mit einem Alkoholgehalt von 50% und mehr offenbart werden.

Optional werden hier mindestens ein Emulgator b.) mit einem HLB - Wert von größer 9 und/oder Rheologiemodifizierer d.) zur Stabilisierung oder zur Veränderung der Viskosität eingesetzt.

Die Zubereitungen zeigen ein homogenes Erscheinungsbild, was erfindungsgemäß dadurch gekennzeichnet ist, dass sich keine Ausfällungen, Phasenbildungen oder Kristallbildung zeigen.

Die Zubereitungen können sowohl transparent, beispielsweise als Mikroemulsion, alkoholische Lösung, als auch transluzent, z.B. als PIT-Emulsion oder opak, z.B. als Makroemulsion, formuliert werden.

Darüber hinaus konnte überraschend festgestellt werden, dass auch die Auswahl der Ölkomponente einen entscheidenden Einfluss auf die Lagerstabilität, insbesondere von Makroemulsionen, besonders bei thermischer Belastung, hat. Besonders bewährt haben sich erfindungsgemäß Triglyceride.

Bevorzugte Triglceride sind zu wählen aus der Gruppe Acetic/Linoleic/Palmitic Triglyceride, C8-12 Acid Triglyceride, C12-18 Acid Triglyceride, C18-36 Acid Triglyceride, Capric/Lauric/Myristic/Oleic Triglyceride, Caprylic/Capric/Lauric Triglyceride, Caprylic/Capric/Linoleic Triglyceride, Caprylic/Capric/Myristic/Stearic Triglyceride, Caprylic/Capric/Palmitic/Stearic Triglyceride, Caprylic/Capric/Stearic Triglyceride, Caprylic/Capric/Succinic Triglyceride, Caprylic/Capric Triglyceride, Caprylic/Capric Triglyceride PEG-4 Esters, C10-40 Isoalkyl Acid Triglyceride, Cod Liver/Mink/Tallow Triglyceride, C10-18 Triglycerides, C10-18 Triglycerides Polyglyceryl-3 Esters Phosphates, Docosahexenoic/Docosapentenoic/Oleic/Palmitic Triglyceride, Hydrogenated C12-18 Triglycerides, Jojoba Oil/Caprylic/Capric Triglyceride Esters, Lauric/Palmitic/Oleic Triglyceride, Mustelic/Palmitic Triglyceride, Oleic/Linoleic Triglyceride, Oleic/Palmitic/Lauric/Myristic/Linoleic Triglyceride, Palmitic/Stearic Triglyceride und Ricinoleic/Caproic/Caprylic/Capric Triglyceride.

Bevorzugt zu wählen sind mittelkettige Triglyceride, bevorzugt das Lipid Caprylic/Capric Triglyceride.

So wurde anhand des Austauschs der Ölkomponente PPG-15 Stearylether gegen Caprylic/Capric Triglyceride eine weiter stabilisierte Zubereitung erhalten. Triglyceride sind demnach für die Stabilität, gerade unter thermischer Belastung, besonders förderlich.

Ebenfalls überraschend zeigten sich die erfindungsgemäß verwendeten Zubereitungen umfassend ein oder mehrere Parfumbestandteile auch hinsichtlich der Parfumstabilität verbessert. D.h. es ist ein längerer Parfumeffekt bemerkbar gegenüber Zubereitungen ohne erfindungsgemäße Zusatzstoffe b. (nichtionische Emulgatoren), c. (kationische Emulgatoren) oder d. (Rheologiemodifizierer).

Eine Zersetzung des Parfums findet nicht statt.

Als besonders bevorzugte, weil weiter stabilisierend und verstärkt desodorierend, haben sich die Parfumkomponenten Terpin-4-ol (CAS 562-74-3), Linalool (78-70-6), Citral, Geraniol, Neroll, Perillaldehyde, $\alpha$-Terpineol, Thymol, Eugenol, Citronellal, Terpinyl Acetate, Citronellol,, ß-Pinene, Benzaldehyde, 4-Methylbenzaldehyde, Heliotropine, Vanillin, 3-Hydroxy-4- methoxybenzaldehyde (Isovanillin), Geranium oil, Peppermint oil, Rose oil, Cinnamon leaf oil, Fucus oil, Clove bud oil, Clove leaf oil, Palmarosa oil, Citrus oil, Tepene fraction of citrus oil, Orange oil, Terpene fraction of orange oil, Cuminic alcohol, Perilla alcohol, Cinnamic alcohol, Limonene, Dihydroterpineol, Citral, Eucalyptol und Eugenol erwiesen.

Die Zubereitungen umfassen bevorzugt keine antitranspirant-wirksamen Substanzen, insbesondere keine Aluminiumsalze, wie Aluminiumchlorohydrate.

Bei der Methode der Molmassenbestimmung handelt es sich um eine GPC mit wässrigem Eluentensystem, kalibriert mit Dextran/Pullulan-Standards.

Methodeninformation

Kalibrationsart: Conventional
Int. Standard - K: 50,00 ml
Int. Standard - M: 0,00 ml

Probenkonzentration: 3,00 g/l
Injektvolumen: 50 μl
Eluent: 0.1 M NaCl / 0.1% TFA
Flussrate: 1,000 ml/min
Säulen: PSS Novema 10μm 30,300,1000
Temperatur: 25,0 °C
GPC-Anlage: PG13

Nachfolgende Vergleichsuntersuchungen zeigen die bemerkenswerten Vorteile der Zubereitungen.

1) Stabilisierung von Mikroemulsionsgelen

In Standard-Mikroemulsionen, wie sie beispielsweise in WO 1998015254 A1 beschrieben sind, wird Polyquaternium-16 eingearbeitet und so die Zubereitung durch ein Verhältnis von Emulgator zu Co-Emulgator stabilisiert. Erstaunlicherweise wurde gegenüber den bekannten Zubereitungen, die C1-C6-Alkoholen umfassen, ohne diese C1-C6 Alkohole dennoch eine antimikrobielle Wirksamkeit erreicht, wie in den angefügten Untersuchungen dargelegt ist.
Die Beispiele 3 bis 6 sind nicht Teil der Erfindung.

| INCI: O/W Emulsion | 1 | 2 | 3 | 4 | 5 | 6 | 7 |
|---|---|---|---|---|---|---|---|
| Persea Gratissima Oil | 0,5 | 0,1 | 0 | 0,1 | 0 | 0,1 | 0,1 |
| Pionier 2076 (Paraffinum Liquidum; Hansen & Rosenthal) | 4 | 3 | 0 | 3 | 2,8 | 0 | 3 |
| Tegin ISO (Glyceryl Isostearate; Evonik Industries) | 2,0 | 2,2 | 2,4 | 2,4 | 1,5 | 2,1 | 2,2 |
| Tego Alkanol IC 20 (Isoceteth-20; Evonik Industries) | 4,2 | 5 | 4,8 | 4,7 | 3,65 | 5 | 5 |
| Cetiol OE (Dicaprylyl Ether, BASF Personal Care and Nutrition) | 0 | 0 | 3 | 0 | 0 | 2 | 0 |
| Glycerin | 0 | 2 | 0 | 0 | 0 | 2 | 2 |
| Trisodium EDTA | 0,5 | 0,2 | 0,5 | 0,5 | 0,2 | 0,2 | 0,2 |
| Octopirox (Piroctone Olamine; Clariant) | 0,05 | 0,05 | 0,05 | 0,05 | 0,05 | 0,05 | 0,05 |
| Parfum | q.s. | q.s | q.s | q.s | q.s | q.s | q.s |
| Butylene Glycol | 4 | 3 | 3 | 3 | 3 | 3 | 3 |
| Kessco PEG 6000 DS HV (PEG-150 Distearate; Italmatch Chemical) | 0,5 | 0,75 | 0,7 | 0,7 | 1,2 | 0,7 | 0,85 |
| Luviquat Excellence (Polyquaternium-16; BASF) | 1,5 | 2,5 | 0 | 0 | 0 | 0 | 2,5 |
| Luviquat FC 550 (Polyquaternium-16; BASF) | 0 | 0 | 1,25 | 4 | 0 | 0 | 0 |
| Luviquat FC 370 (Polyquaternium-16; BASF) | 0 | 0 | 0 | 0 | 3,5 | 0 | 0 |
| Luviquat Style (Polyquaternium-16; BASF) | 0 | 0 | 0 | 0 | 0 | 3,5 | 0 |
| Aqua | ad 100 | | | | | | |
| Resultat: Homogene Zubereitung | | | | | | | |

Homogen bedeutet dabei keinerlei Ausfällungen, Agglomerationen, Phasenbildungen. Die Emulgatorsysteme weisen einen HLB - Wert im Bereich von 10 bis 14 auf.

2) Stabilisierung von Makroemulsionen - Die Beispiele 9 bis 11 sind nicht Teil der Erfindung

| INCI O/W Emulsion | 8 | 9 | 10 | 11 |
|---|---|---|---|---|
| Persea Gratissima Oil | 0,1 | 0,1 | 0,25 | 0,2 |
| Cetiol E ( PPG-15 Stearyl Ether; BASF Personal Care and Nutrition) | 4 | 4 | 3 | 4 |
| Tego Alkanol S 21 (Steareth-21; Evonik Industries) | 2,5 | 2,3 | 2,0 | 2,6 |

(fortgesetzt)

| INCI O/W Emulsion | 8 | 9 | 10 | 11 |
|---|---|---|---|---|
| Tego Alkanol S2 (Steareth-2; Evonik Industries) | 1,5 | 1,7 | 2,0 | 1,7 |
| Parfum | q.s. | q.s. | q.s. | q.s. |
| Trisodium EDTA | 0,3 | 0,3 | 0,3 | 0,3 |
| Piroctone Olamine (Octopirox; Clariant) | 0,05 | 0,05 | 0,05 | 0,05 |
| Natrosol 250 HHX pharm (Hydroxyethylcellulose; Ashland Aqualon Functional Ingredients) | 0,35 | 0,45 | 0,45 | 0,35 |
| Luviquat Excellence (Polyquaternium-16; BASF) | 1,5 | 0 | 0 | 0 |
| Luviquat FC 550 (Polyquaternium-16; BASF) | 0 | 2 | 0 | 0 |
| Luviquat FC 370 (Polyquaternium-16; BASF) | 0 | 0 | 1 | 0 |
| Luviquat Style (Polyquaternium-16; BASF) | 0 | 0 | 0 | 1,5 |
| Aqua | ad 100 | | | |
| Resultat: Homogene Zubereitung | | | | |

Die Beispiele 13 und 15 sind nicht Teil der Erfindung.

| INCI O/W Emulsion | 12 | 13 | 14 | 15 | 16 |
|---|---|---|---|---|---|
| Persea Gratissima Oil | 0,1 | 0,1 | 0,1 | 0,2 | 0,3 |
| Myritol 312 (Caprylic/Capric Triglyceride; BASF Personal Care and Nutrition) | 4 | 4 | 3 | 4 | 3 |
| Tego Alkanol S 21 (Steareth-21; Evonik Industries) | 0,5 | 1 | 2 | 2 | 4 |
| Tego Alkanol S2 (Steareth-2; Evonik Industries) | 5 | 4 | 3,5 | 3 | 2 |
| Parfum | q.s. | q.s. | q.s. | q.s. | q.s. |
| Trisodium EDTA | 0,3 | 0,3 | 0,3 | 0,3 | 0,3 |
| 100981 Benzyl alcohol EMPROVE exp Ph Eur,BP,JP,NF ( Benzyl Alcohol; Merck) | 0,3 | 0,2 | 0,3 | 0,5 | 0,25 |
| Phenoxyethanol R + (Phenoxyethanol; Univar) | 0,7 | 0,5 | 0,5 | 0,2 | 0,5 |
| Glycerol EP vegetable (Glycerin, Spiga Nord) | 5 | 8 | 5 | 4 | 5 |
| Luviquat Excellence (Polyquaternium-16; BASF) | 1,5 | 0 | 2,5 | 0 | 1,5 |
| Luviquat FC 550 (Polyquaternium-16; BASF) | 0 | 2 | 0 | 0 | 0 |
| Luviquat FC 370 (Polyquaternium-16; BASF) | 0 | 0 | 0 | 1 | 0 |
| Luviquat Style (Polyquaternium-16; BASF) | 0 | 0 | 0 | 1,5 | 0 |
| Aqua | ad 100 | | | | |
| Resultat: Homogene Zubereitung | | | | | |
| HLB | 5,85 | 7,02 | 8,7 | 9,14 | 11,85 |

Die dargestellten Makroemulsionen sind mit einem HLB des Emulgatorsystems von 4 bis 12 stabil, d.h. keine Ausfällungen, kein olfaktorischen Veränderungen etc.

3) Stabilisierung in alkoholischer Lösung

Für die Stabilisierung von Zubereitungen umfassend Polyquaternium Verbindungen ist nicht zwingend eine Emulsion erforderlich. Auch durch Einsatz von PQ-16 in einer optional mit einem Rheologiemodifizierer verdickten alkoholischen Lösung und mindestens einem Emulgator, bevorzugt nichtionisch mit einem HLB von größer 9, wird eine stabile Zubereitung erhalten.

Überraschend wird dadurch eine Stabilisierung schon mit einem Alkoholgehalt unter 50% erreicht, bevorzugt 45% bzw. 30%, wohingegen im Stand der Technik (US 20070184016 A1, WO2007095008 A2) der Alkoholgehalt mindestens 50% beträgt.

| INCI: Alkoholische Lösung - Die Beispiele 17 bis 19 und 22 bis 23b sind nicht Teil der Erfindung | 17 Roll-on | 18 Zerstäuber | 19 Roll-on | 20 Zerstäuber | 21 Roll-on | 22 Zerstäuber | 23 Roll-on | 23b Zerstäuber |
|---|---|---|---|---|---|---|---|---|
| Citric Acid | 0,05 | 0,1 | 0,05 | 0,05 | 0,05 | 0,1 | 0,05 | 0,1 |
| Natrosol 250 HHX pharm (Hydroxyethylcellulose; Ashland Aqualon Functional Ingredients) | 0,2 | 0 | 0,3 | 0 | 0,35 | 0 | 0,2 | 0 |
| Eutanol G (Octyldodecanol; BASF Personal Care and Nutrition) | 0,1 | 0,1 | 0,1 | 0,1 | 0,1 | 0,1 | 0,1 | 0,1 |
| Ethanol | 30 | 45 | 30 | 45 | 30 | 45 | 30 | 45 |
| Parfum | q.s. | q.s. | q.s. | q.s. | q.s. | q.s. | q.s. | q.s. |
| Polyglykol 400 (PEG-8; Clariant) | 1,0 | 0 | 1,0 | 0 | 2,0 | 0 | 1,0 | 0 |
| Cremophor RH 410 ( PEG-40 Hydrogenated Castor Oil; BASF) | 1,5 | 1 | 1,5 | 1 | 2,0 | 1 | 1,5 | 1 |
| Luviquat Excellence (Polyquaternium-16; BASF) | 0 | 0 | 0 | 2,5 | 2,5 | 0 | 0 | 0 |
| Luviquat FC 550 (Polyquaternium-16; BASF) | 0 | 2 | 3 | 0 | 0 | 0 | 0 | 0 |
| Luviquat FC 370 (Polyquaternium-16; BASF) | 4 | 0 | 0 | 0 | 0 | 3 | 0 | 0 |
| Luviquat Style (Polyquaternium-16; BASF) | 0 | 0 | 0 | 0 | 0 | 0 | 2 | 3 |
| Aqua | Ad 100 | | | | | | | |
| Resultat: Homogene Zubereitung | | | | | | | | |

In anderen Zubereitungen ist ein homogenes Erscheinungsbild bei Anwesenheit von Polyquaternium-16 nicht möglich, wie die nachfolgenden Rezepturbeispiele 25 bis 30 zeigen.

Liegt wie hier kein optimales Verhältnis von Emulgator zu Co-Emulgator vor bzw. liegt der HLB des Emulgators bzw. des Solubilisierers bzw. der Kombination der Emulgatoren nicht im optimalen Bereich, sind Agglomerationen, Phasenbildungen bzw. eine Destabilisierung der Formulierung zu beobachten.

Die Beispiele 26 bis 28 sind nicht Teil der Erfindung.

| INCI: O/W Emulsion | 25 | 26 | 27 | 28 |
|---|---|---|---|---|
| Tegin VS (Glyceryl Stearate SE; Evonik Industries) | 2,0 | 1,8 | 2,0 | 2,1 |
| Lanette E granules (Sodium Cetearyl Sulfate; BASF Personal Care and Nutrition) | 0,5 | 0,7 | 0,5 | 0,4 |
| Parfum | q.s. | q.s. | q.s. | q.s. |
| Glycerin | 7 | 7 | 7 | 7 |
| Sodium Hydroxide | 0,5 | 0,5 | 0,5 | 0,5 |
| Phenoxyethanol | q.s. | q.s. | q.s. | q.s. |
| Methylisothiazolinone | q.s. | q.s. | q.s. | q.s. |
| Carbopol 981 (Carbomer; Lubrizol Advanced Materials) | 0,25 | 0,3 | 0,25 | 0,25 |
| Alcohol Denat. | 6 | 5 | 8 | 7 |
| Luviquat Excellence (Polyquaternium-16; BASF) | 1,5 | 0 | 0 | 0 |
| Luviquat FC 550 (Polyquaternium-16; BASF) | 0 | 2 | 0 | 0 |
| Luviquat FC 370 (Polyquaternium-16; BASF) | 0 | 0 | 1 | 0 |
| Luviquat Style (Polyquaternium-16; BASF) | 0 | 0 | 0 | 1,5 |
| Aqua | ad 100 | | | |
| Resultat | Agglomeration | | | |

Agglomerationen, Phasenbildungen bzw. eine Destabilisierung der Formulierung sind zu beobachten. Bei folgenden opake Formulierungen treten bei thermischer Belastung Instabilitäten auf:

| INCI O/W Emulsion | 29 | 30 |
|---|---|---|
| Persea Gratissima Oil | 0,1 | 0,1 |
| Cetiol E ( PPG-15 Stearyl Ether; BASF Personal Care and Nutrition) | 4 | 0 |
| Benzoate DE C12/C15 (C12-15 Alkyl Benzoate; Stearinerie Dubois) | 0 | 3 |
| Tego Alkanol S 21 (Steareth-21; Evonik Industries) | 1,5 | 1,2 |
| Tego Alkanol S2 (Steareth-2; Evonik Industries) | 2,5 | 2,8 |
| Parfum | q.s. | q.s. |
| Trisodium EDTA | 1,5 | 1,5 |
| 100981 Benzyl alcohol EMPROVE exp Ph Eur,BP,JP,NF ( Benzyl Alcohol; Merck) | 0,3 | 0,2 |
| Phenoxyethanol R + (Phenoxyethanol; Univar) | 0,7 | 0,5 |
| Luviquat Excellence (Polyquaternium-16; BASF) | 3 | 2 |
| Aqua | ad 100 | |
| Resultat: Phasentrennung | | |

Überraschenderweise zeigen die Polyquaternium-16 Polymere gegenüber den Bakterien Staphylococcus epidermidis und Corynebacterium jeikeium eine antimikrobielle Wirksamkeit und damit eine Deo-wirkung.

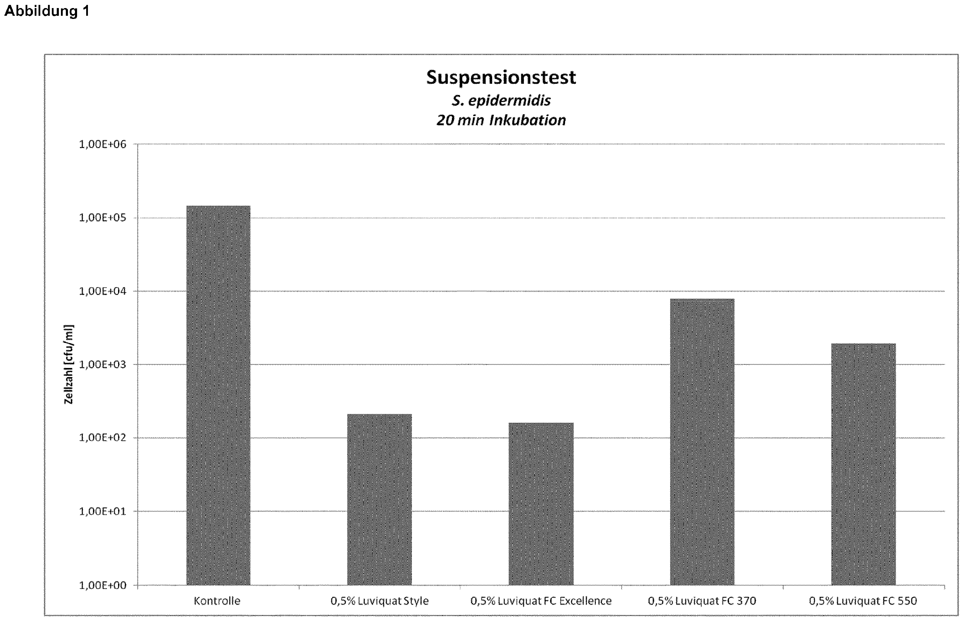

Im einem Suspensionstest (s. Abbildung 1) wurden verschiedene PQ-16 Varianten untersucht. Die erfindungsgemäßen PQ-16 Verbindungen zeigen eine antimikrobielle Wirksamkeit und Deo-wirkung.

Andere antimikrobielle Stoffe, wie beispielsweise Triclosan, der durchaus als antimikrobieller Stoff bekannt ist, zeigt gegen diese Deo-relevanten Bakterien Staphylococcus epidermidis und Corynebacterium jeikeium keine bzw. nicht ausreichende Wirksamkeit, wie auch in J. Soc. Cosmet. Chem., 38, 223-231 (July/August 1987) - Efficacy of the antimicrobial agent triclosan in topical deodorant products - gezeigt wurde.

Es zeigt sich, dass Triclosan keine antimikrobielle Wirksamkeit in der Achsel gegenüber dem ethanolischem Aerosolspray zeigt, zwar eine geringe Wirksamkeit nach 12h im Sniff-Test, aber keine Wirksamkeit nach 24h im Sniff-Test.

Erfindungsgemäße Zubereitungen zeigen sowohl nach Stunden aus beispielsweise einem ethanolischem Pumpspray (45% Ethanol) eine deutliche antimikrobielle Wirksamkeit in der Achsel und eine 24h Wirksamkeit gegenüber dem Vehikel im Sniff-Test.

Es ist daher nicht allein aus der Kenntnis einer allgemeinen antimikrobiellen Wirksamkeit gleichzeitig auf eine gute bzw. überhaupt vorhandene desodorierende Wirksamkeit zu schließen.

Für eine Deowirksamkeit müssen die entscheidenden Bakterien, wie Bakterien Staphylococcus epidermidis, signifikant reduziert werden.

Dazu wurden Suspensionstests wie folgt durchgeführt:

Das Bakterium *S. epidermidis (ATCC 12228)* wurde aus einem nach EN 12353 angelegten Cryoröhrchen auf einer entsprechenden Agarplatte (TSA-Agar) ausgestrichen und bei 37°C 24 Stunden bebrütet. Von den gewachsenen Bakterien wurde eine zweite Passage angelegt, die im Suspensionstest eingesetzt wurde.

Eine Impföse der Bakterien wurde zu 10mL LB-Medium (n.G. Bertani) und 5g Glasperlen mit 4mm Durchmesser gegeben und 3 Minuten gevortext. Anschließend wurde diese Bakteriensuspension auf eine OD (optischen Dichte) von 0,08 eingestellt. Die eingestellte Bakteriensuspension wurde dann 1:20 mit LB-Medium verdünnt, um eine Keimzahl von ungefähr $5*10^5$ zu gewährleisten. Diese Bakteriensuspension wurde anschließend für den Test eingesetzt.

Der Suspensionstest wurde per Hand durchgeführt. Hierzu wurden 900µL der zu testenden Wirkstofflösungen bzw. im Falle der Kontrollen 900 µl Phosphatpuffer in Eppendorfgefäßen vorgelegt. Anschließend wurden 100 µl der Bakteriensuspension hinzugegeben und gevortext. Zum Zeitpunkt to wurde eine Probe aus den Kontrollen entnommen. Die Eppendorfgefäße wurden nun im Thermoschüttler bei 37 °C inkubiert. Nach 20, 60 und 180 Minuten bei 37°C und 1200rpm wurden aus den Eppendorfgefäßen im Thermoschüttler jeweils 100µL entnommen und zu 900µL LB-Medium pipettiert ($10^{-1}$-Verdünnung). Diese Verdünnungen wurden mittels Spiralplatter auf Agarplatten ausplattiert. Eine Ausnahme stellt hier die Kontrolle dar, die nochmal 1:100 verdünnt wurde ($10^{-3}$-Verdünnug). Bei den Kontrollen wurden beide Verdünnungen ausplattiert, einschließlich des Zeitpunkts T=0 Minuten. Zum Schluss wurden die Platten bei 37°C 24 Stunden bebrütet und anschließend mit Hilfe des Countermaten ausgewertet.

In der Auswertung wurde der Reduktionsfaktor bestimmt. Der Reduktionsfaktor ist der Faktor, um den die Zellzahl (CFU) der Testsubstanz im Vergleich zur Kontrolle reduziert wird.

$$\frac{CFU(Kontrolle)}{CFU\ (Testsubstanz)} = Reduktionsfaktor$$

| Wirkstofflösung | Reduktionsfaktor 20 Min |
| --- | --- |
| 0,5% Luviquat FC Excellence | 919 |
| 0,5% Luviquat FC 550 | 77 |
| 0,5% Luviquat FC 370 | 19 |
| 0,5% Luviquat Style | 689 |

Die angegeben Prozentgehalte beziehen sich auf den Gewichtsanteil an Aktivsubstanz, also ohne Lösemittel Wasser, im Phosphatpuffer.

Die Rohstoffe wurden in Phosphatpuffer gelöst.

Phosphatpuffer umfasst:

A) Kaliumdihydrogenphosphat 9,078 g/L
B) Dinatriumhydrgenphosphat *2 $H_2O$ 11,876 g/L
122 mL A zu 363 mL B = Gebrauchslösung

Es zeigte sich, dass die erfindungsgemäßen Polyquaternium Polymere gemäß Anspruch 1 zu einer entscheidenden Bakterienreduktion führen und somit eine Deowirksamkeit zeigen.

Als desodorierend wirksam wird ein Reduktionsfaktor von mindestens 10 angesehen.

Insbesondere Polyquaternium-16 Polymere mit einem VP/QVI Verhältnis im Bereich von 5 zu 95 (Luviquat Excellence) zeigen eine besonders gute desodorierende Wirkung, die so nicht zu erwarten war (Reduktionsfaktor 919).

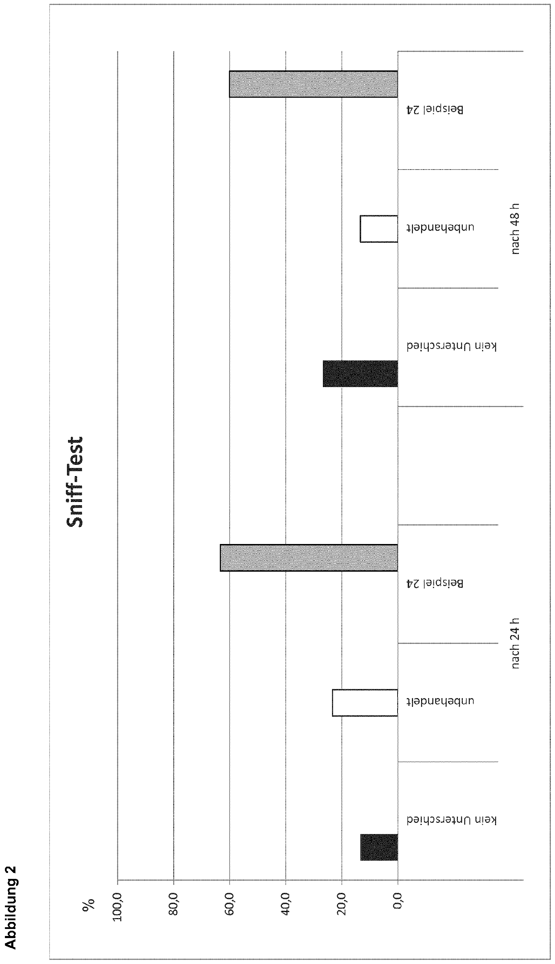

In einem Sniff-Test (s. Abbildung 2) zeigte sich ebenso eine signifikante Geruchsreduktion aus einem unparfümierten Mikroemulsionsgel (Beispiel 24).

1% Polyquaternium-16 erzielt nach einer Einmalapplikation eine deutliche Geruchsreduktion gegenüber der unbehandelten Achsel.

Der Sniff Test (SNK) dient der Bestimmung einer i.d.R. 48-stündigen desodorierenden Wirksamkeit von kosmetischen Formulierungen nach einmaliger Anwendung. Die Anwendung des Testproduktes und die Messung der Wirksamkeit erfolgen in dem verbraucherrelevanten Areal, der Achsel. Zielgröße ist die Geruchsintensität des Achselschweißes.

Messung

Die Bestimmung der deodorierenden Wirksamkeit wird über die Verwendung valider und anerkannter Messmethoden und Beurteilungsverfahren realisiert.

Das Studiendesign ist so ausgelegt, dass deodorierende Wirksamkeiten wie folgt nachgewiesen werden können:

- zumindest einfach verblindet und randomisiert in Bezug auf die Prüfmuster und offen für die unbehandelte Kontrolle
- Vergleich zur unbehandelten Kontrolle -> Wirknachweis für Wirkstoff & Formulierung.

Die Messung erfolgt durch eine subjektive, olfaktorische Beurteilung der Intensität des Achselschweißes durch ein Sniffer-Panel (weibliche und männliche Probanden). Die Beurteilung der Geruchsintensität des Achselschweißes erfolgt i.d.R. für eine vergleichende Beurteilung der Geruchsproben nach den absoluten Skalenwerten von 0 (kein Schweißgeruch) bis 5 (sehr starker Schweißgeruch).

Geprüft wird ein codiertes Prüfmuster (Codierung gemäß WI 7 MAT 100) im kontralateralen Vergleich gegen die unbehandelte Kontrolle.

Nach 24h konnte bei 63,3% der Probanden in der mit der erfindungsgemäßen Zubereitung behandelten Achsel eine signifikante Reduktion des Schweißgeruchs durch das Sniffer-Panel wahrgenommen werden. Bei 13,3% der Probanden konnte kein Unterschied festgestellt werden. Bei 23,3% der Probanden wurde die unbehandelte Achsel besser bewertet (s. Abbildung 2).

1% Polyquaternium-16 erzielt nach einer Einmalapplikation daher eine deutliche Geruchsreduktion gegenüber der unbehandelten Achsel.

Gleiche Tests wurden auch zur Ermittlung des Deowirksamkeit auf Textilien durchgeführt.

In einem Test wurde die Deowirksamkeit gegen das Bakterium S. epidermidis (ATCC 12228) ermittelt. Die Untersuchungen erfolgte entsprechend der Bestimmung der antibakteriellen Wirkung antibakteriell behandelter Erzeugnisse (ISO 20743:2007).

Die wie zuvor dargestellte Bakteriensuspension wurde anschließend für den Test eingesetzt.

Hierzu wurden 30µL der zu testenden Wirkstofflösungen bzw. im Falle der Kontrollen 30 µl Wasser auf Baumwoll- und Polyester Stanzen pipettiert und 1 h trocknen gelassen. Anschließend wurde 50 µl der Bakteriensuspension hinzugegeben. Die Textilproben wurden dann im Liconic Inkubator bei 37°C und 90% Luftfeuchtigkeit inkubiert. Nach 60 Minuten wurden die Textilproben in Eppendorfgefäße überführt, die 1 ml Neutralisationsmedium enthalten. Nach 1 Minute im Thermoschüttler und unter Intervallschüttlung (5 Sekunden/ 3 Sekunden) wurden die Textilproben entfernt und die Lösung mittels Spiralplatter auf Agarplatten ausplattiert. Zum Schluss wurden die Platten bei 37°C 24 Stunden bebrütet und anschließend mit Hilfe des Countermaten ausgewertet.

In der Auswertung wurde der Reduktionsfaktor bestimmt. Der Reduktionsfaktor ist der Faktor, um den die Zellzahl (CFU) der Testsubstanz im Vergleich zur Kontrolle reduziert wird.

$$\frac{CFU(Kontrolle)}{CFU\ (Testsubstanz)} = Reduktionsfaktor$$

| Wirkstofflösung (50 µg/cm$^2$ Textil) | Reduktionsfaktor 20 Min |
|---|---|
| Alexidine (1,1'-(1,6-Hexanediyl)bis{2-[N'-(2-ethylhexyl)carbamimidoyl]guanidine}) | 6 |

(fortgesetzt)

| Wirkstofflösung (50 μg/cm² Textil) | Reduktionsfaktor 20 Min |
|---|---|
| Polyquaternium-37 (AMP019) | 12 |
| Polyquaternium-16 (Luviquat Excellence) | 1335 |
| Polyaminopropyl Biguanide (Cosmocil CQ) | 109 |

Erstaunlich zeigte sich auch hier, dass antimikrobiell bekannte Stoffe (Alexidine) keinerlei desodorierende Wirksamkeit aufweisen.

Erstaunlich ist jedoch vor allem, dass Polyquaternium-16 Polymere auf Textilien aufgebracht zu einer beachtenswerten Deowirkung führen.

Es zeigte sich, dass insbesondere Polyquaternium-16 Polymere mit einem VP/QVI Verhältnis im Bereich von 5 zu 95 (Luviquat Excellence) eine besonders gute desodorierende Wirkung zeigen (Reduktionsfaktor von 1335).

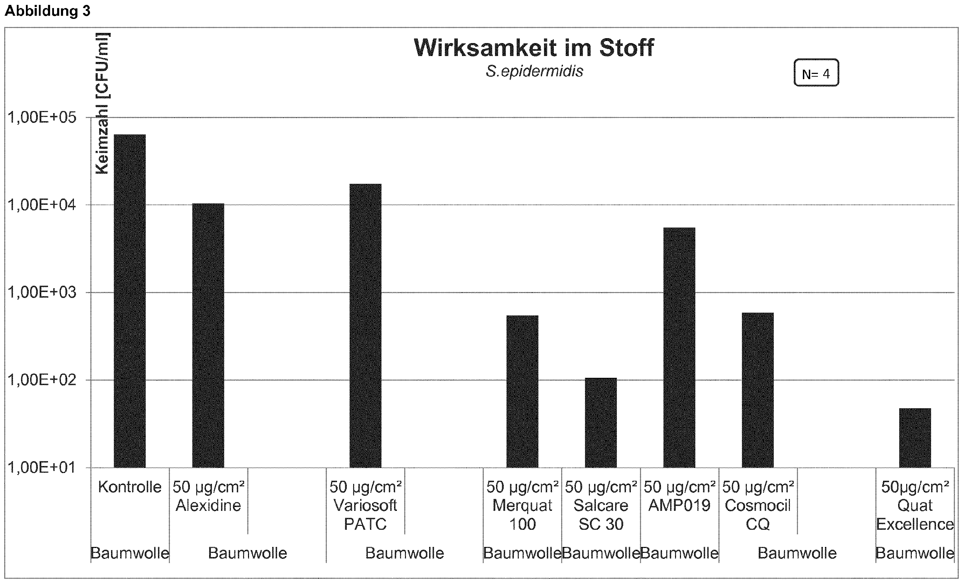

Die Keimzahlreduktion unterschiedlicher antimikrobieller Stoffe auf Baumwolle zeigt Abbildung 3. Die angegebenen Konzentrationswerte beziehen sich auf Gewicht pro Quadratzentimeter an reinem Wirkstoff (Aktivmenge).

Es wurden getestet ein Verdicker Palmitamidopropyltrimonium Chloride (Variosoft PATC), Polyaminopropyl Biguanide (Cosmocil CQ), Polyquaternium-6 (Merquat 100, Salcare SC 30), Polyquaternium 37 (AMP019) und PQ-16 (Luviquat Excellence).

PQ-16 zeugte gegenüber allen anderen getesteten Polyquaternium Polymere und den Vergleichszubereitungen eine signifikant bessere Keimreduktion.

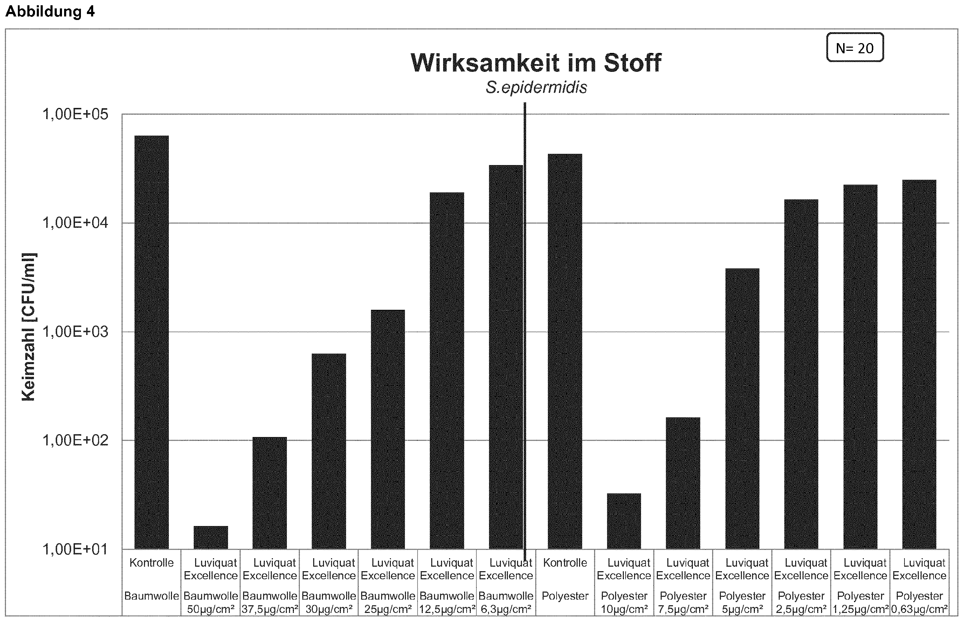

In einer weiteren Untersuchung wurde der Einfluss der Konzentration der Polyquaternium Polymere auf die Deowirkung auf Textilien untersucht (Abbildung 4).

Es wurde dazu wie zuvor dargestellt die Keimzahlreduktion unterschiedlich konzentrierter PQ-16 Polymeren (Luviquat Excellence) auf Baumwolle und Polyester Textilien ermittelt. Die angegebenen Konzentrationswerte beziehen sich auf Gewicht pro Quadratzentimeter an reinem Wirkstoff (PQ-16).

In den Untersuchungen zeigten sich auch untere Wirkgrenzen, die bei Aufbringen auf Baumwolle bei $12,5 \mu g/cm^2$ und bei Polyester bei $2,5 \mu g/cm^2$ liegen. Unterhalb dieser Auftragungsmenge ist keine Deowirkung auf Textilien mehr zu beobachten.

In einem weiteren Sniff-Test wurde die Deowirksamkeit von PQ-16 auf Textilien untersucht.

Hierzu wurden Baumwoll-Pads mit einer Konzentration von 5 mg/cm² Tränkungsmedien getränkt, 14h getrocknet, in die T-Shirts geklettet und 8h getragen.

Die getesteten Tränkungsmedien umfassten einmal Verum bestehend aus 3,75% Luviquat Excellence (40%), 45 % Ethanol, 51,25% Wasser und zum anderen das Kontrollmedium (Vehikel) bestehend aus 45% Ethanol und 55% Wasser.

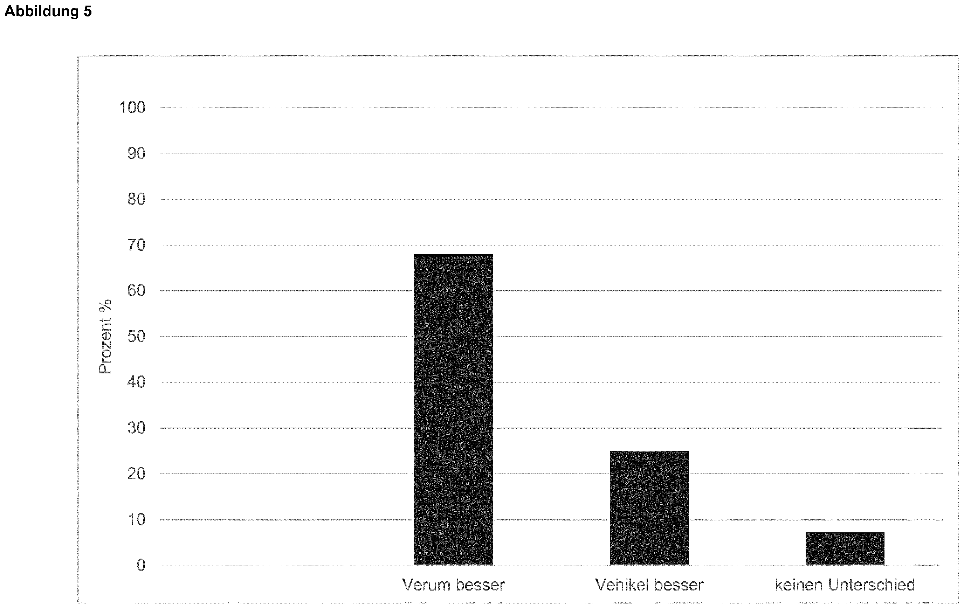

Entsprechend den oben ausgeführten Sniff-Test Bedingungen ergaben sich folgende Resultate wie sie in Abbildung 5 dargestellt sind.

Nach 8 Stunden konnte bei 67,9% der Probanden in der mit der erfindungsgemäßen Zubereitung behandelten Achsel eine signifikante Reduktion des Schweißgeruchs durch das Sniffer-Panel wahrgenommen werden. Bei 7,1% der Probanden konnte kein Unterschied festgestellt werden. Bei 25% der Probanden wurde die unbehandelte Achsel besser bewertet.

Polyquaternium-16 Polymere gemäß Anspruch 1 sind demnach für die Desodorierung von Textilien geeignet.

In der Anwendung der Zubereitungen mit PQ-16 Polymeren auf oder durch die Kleidung bieten sich vorzugsweise Polyquaternium Polymere enthaltene Zubereitungen an, die als Aerosol- oder Pumpspray konzipiert sind.

Zubereitungen sind bevorzugt O/W-Emulsionen, Makroemulsionen, Mikroemulsionen, alkoholische Zubereitungen, PIT-Emulsionen und Hydrodispersionen.

Die kosmetischen oder dermatologischen Zubereitungen können ferner kosmetische Hilfsstoffe und Wirkstoffe enthalten, wie sie üblicherweise in solchen Zubereitungen verwendet werden, z. B. Wirkstoffe, Konservierungsmittel, Konservierungshelfer, Bakterizide, Lipide, Substanzen zum Verhindern des Schäumens, Farbstoffe und Farbpigmente, Verdickungsmittel, anfeuchtende und/oder feuchthaltende Substanzen oder andere übliche Bestandteile einer kosmetischen oder dermatologischen Formulierung wie Polyole, Polymere, Schaumstabilisatoren, organische Lösungsmittel oder Silikonderivate, sofern der Zusatz die geforderten Eigenschaften hinsichtlich der Stabilität nicht beeinträchtigen oder ausgeschlossen sind.

Die vorstehenden und nachfolgenden Beispiele veranschaulichen Zubereitungen. Die Zahlenangaben sind Gewichtsanteile bezogen auf die Gesamttasse der Zubereitung, sofern nichts anderes angegeben ist. Die Anteilsangaben beziehen sich auf die eingesetzten Rohstoffe bzw. Handelsprodukte. Der Aktivgehalt an PQ-Polymeren ergibt sich dann aus den Herstellerangaben, wie

PQ-16

- Luviquat Excellence (38-42% PQ-16 in Wasser)
- Luviquat FC 550 (38-42% PQ-16 in Wasser)
- Luviquat FC 370 (38-42% PQ-16 in Wasser)
- Luviquat Style (19-21% PQ-16 in Wasser)

Beispiele

1) Mikroemulsionsgele - Die Beispiele 31a bis 34 sind nicht Teil der Erfindung

| INCI: O/W Emulsion | 24 | 32 | 31a | 32a | 33 | 34 | 35 |
|---|---|---|---|---|---|---|---|
| Persea Gratissima Oil | 0,5 | 0,1 | 0 | 0,1 | 0 | 0,1 | 0,1 |
| Pionier 2076 (Paraffinum Liquidum; Hansen & Rosenthal) | 4 | 3 | 0 | 3 | 2,8 | 0 | 3 |
| Tegin ISO (Glyceryl Isostearate; Evonik Industries) | 2,2 | 2,2 | 2,4 | 2,4 | 1,5 | 2,1 | 2,2 |
| Tego Alkanol IC 20 (Isoceteth-20; Evonik Industries) | 4,8 | 5 | 4,8 | 4,7 | 3,65 | 5 | 5 |
| Cetiol OE (Dicaprylyl Ether, BASF Personal Care and Nutrition) | 0 | 0 | 3 | 0 | 0 | 2 | 0 |
| Glycerin | 2 | 2 | 0 | 0 | 0 | 2 | 2 |
| Trisodium EDTA | 0,5 | 0,2 | 0,5 | 0,5 | 0,2 | 0,2 | 0,2 |
| Octopirox (Piroctone Olamine; Clariant) | 0,05 | 0,05 | 0,05 | 0,05 | 0,05 | 0,05 | 0,05 |
| Parfum | q.s. | q.s | q.s | q.s | q.s | q.s | q.s |
| Butylene Glycol | 4 | 3 | 3 | 3 | 3 | 3 | 3 |
| Kessco PEG 6000 DS HV (PEG-150 Distearate; Italmatch Chemical) | 0,7 | 0,75 | 0,7 | 0,7 | 1,2 | 0,7 | 0,85 |
| Luviquat Excellence (Polyquaternium-16; BASF) | 2,5 | 2,5 | 0 | 0 | 0 | 0 | 2,5 |
| Luviquat FC 550 (Polyquaternium-16; BASF) | 0 | 0 | 1,25 | 4 | 0 | 0 | 0 |
| Luviquat FC 370 (Polyquaternium-16; BASF) | 0 | 0 | 0 | 0 | 3,5 | 0 | 0 |
| Luviquat Style (Polyquaternium-16; BASF) | 0 | 0 | 0 | 0 | 0 | 3,5 | 0 |
| Aqua | ad 100 | | | | | | |
| Resultat: Homogene Zubereitung | | | | | | | |

2) Makroemulsionen - Die Beispiele 37 bis 39 sowie 41 sind nicht Teil der Erfindung

| INCI O/W Emulsion | 36 | 37 | 38 | 39 | 40 | 41 |
|---|---|---|---|---|---|---|
| Persea Gratissima Oil | 0,1 | 0,1 | 0,25 | 0,2 | 0,1 | 0,1 |
| Cetiol E (PPG-15 Stearyl Ether; BASF Personal Care and Nutrition) | 4 | 4 | 3 | 4 | 2 | 2 |
| Tego Alkanol S 21 (Steareth-21; Evonik Industries) | 2,5 | 2,3 | 2,0 | 2,6 | 3 | 2,8 |
| Tego Alkanol S2 (Steareth-2; Evonik Industries) | 1,5 | 1,7 | 2,0 | 1,7 | 1 | 1,2 |
| Parfum | q.s. | q.s. | q.s. | q.s. | q.s. | q.s. |
| Trisodium EDTA | 1,5 | 1,5 | 1,5 | 1,5 | 1,5 | 1,5 |
| Piroctone Olamine (Octopirox; Clariant) | 0,05 | 0,05 | 0,05 | 0,05 | 0,05 | 0,05 |
| Luviquat Excellence (Polyquaternium-16; BASF) | 1,5 | 0 | 0 | 0 | 2 | 0 |
| Luviquat FC 550 (Polyquaternium-16; BASF) | 0 | 2 | 0 | 0 | 0 | 0 |

(fortgesetzt)

| INCI O/W Emulsion | 36 | 37 | 38 | 39 | 40 | 41 |
|---|---|---|---|---|---|---|
| Luviquat FC 370 (Polyquaternium-16; BASF) | 0 | 0 | 1 | 0 | 0 | 1,5 |
| Luviquat Style (Polyquaternium-16; BASF) | 0 | 0 | 0 | 1,5 | 0 | 0 |
| Aqua | ad 100 | | | | | |
| Resultat: Homogene Zubereitung | | | | | | |

3) Alkoholische Zubereitungen - Die Beispiele 44 bis 49 sind nicht Teil der Erfindung

| INCI: Alkoholische Lösung | 42 | 43 | 44 | 45 | 46 | 47 | 48 | 49 |
|---|---|---|---|---|---|---|---|---|
| Citric Acid | 0,05 | 0,1 | 0,05 | 0,1 | 0,05 | 0,1 | 0,05 | 0,1 |
| Diammonium Citrate | 0 | 0,3 | 0 | 0 | 0 | 0,3 | 0 | 0 |
| Natrosol 250 HHX pharm (Hydroxyethylcellulose; Ashland Aqualon Functional Ingredients) | 0,2 | 0 | 0,3 | 0 | 0,35 | 0 | 0,2 | 0 |
| Eutanol G (Octyldodecanol; BASF Personal Care and Nutrition) | 0,1 | 0,1 | 0,1 | 0,1 | 0,1 | 0,1 | 0,1 | 0,1 |
| Ethanol | 30 | 45 | 30 | 45 | 30 | 45 | 30 | 45 |
| Parfum | q.s. | q.s. | q.s. | q.s. | q.s. | q.s. | q.s. | q.s. |
| Polyglykol 400 (PEG-8; Clariant) | 1,0 | 0 | 1,0 | 0 | 1,0 | 0 | 1,0 | 0 |
| Cremophor RH 410 ( PEG-40 Hydrogenated Castor Oil; BASF) | 1,5 | 1 | 1,5 | 1 | 1,5 | 1 | 1,5 | 1 |
| Luviquat Excellence (Polyquaternium-16; BASF) | 4 | 3,5 | 0 | 0 | 0 | 0 | 0 | 0 |
| Luviquat FC 550 (Polyquaternium-16; BASF) | 0 | 0 | 3 | 2,5 | 0 | 0 | 0 | 0 |
| Luviquat FC 370 (Polyquaternium-16; BASF) | 0 | 0 | 0 | 0 | 3,5 | 3 | 0 | 0 |
| Luviquat Style (Polyquaternium-16; BASF) | 0 | 0 | 0 | 0 | 0 | 0 | 2 | 3 |
| Aqua | Ad 100 | | | | | | | |
| Resultat: Homogene Zubereitung | | | | | | | | |

4) transparente und translucente PIT-Emulsionen - Die Beispiele 50, 52 und 53 sind nicht Teil der Erfindung

| INCI | 50 | 51 | 52 | 53 |
|---|---|---|---|---|
| Eutanol G (Octyldodecanol; BASF Personal Care and Nutrition) | 3 | 0,1 | 0 | 3 |
| Cetiol CC (Dicaprylyl Carbonate; BASF Personal Care and Nutrition) | 1 | 0 | 0 | 1 |
| Cetiol OE (Dicaprylyl Ether, BASF Personal Care and Nutrition) | 0 | 5 | 3 | 0 |
| Cetiol LC (Coco-Caprylate/Caprate, BASF Personal Care and Nutrition) | 0 | 4,5 | 3 | 0 |
| Luviquat Excellence (Polyquaternium-16; BASF) | 0 | 2,5 | 0 | 0 |
| Luviquat FC 550 (Polyquaternium-16; BASF) | 1,5 | 0 | 0 | 0 |
| Luviquat FC 370 (Polyquaternium-16; BASF) | 0 | 0 | 2 | 0 |
| Luviquat Style (Polyquaternium-16; BASF) | 0 | 0 | 0 | 1 |
| Brij IC20N-SO-(AP) (Isoceteth-20; Croda) | 0 | 0 | 4,7 | 0 |
| Tegin ISO Spezial (Glyceryl Isostearate; Evonik Industries) | 2 | 0 | 2,50 | 1,8 |

(fortgesetzt)

| INCI | 50 | 51 | 52 | 53 |
|---|---|---|---|---|
| Rewoderm 66 E 20 (Isosteareth-20; Evonik Industries) | 4 | 0 | 0 | 5,2 |
| Eumulgin B 2 (Ceteareth-20; BASF Personal Care and Nutrition) | 0 | 2 | 2,1 | 0 |
| Emulgade SE-PF (Glyceryl Stearate + Ceteareth-20 + Cetearyl Alcohol + Cetyl Palmitate + Ceteareth-12; BASF Personal Care and Nutrition) | 0 | 3,8 | 3,7 | 0 |
| Glycerin | 3 | 2,6 | 2,2 | 3 |
| Piroctone Olamine (Octopirox; Clariant) | 0,05 | 0,05 | 0,05 | 0,05 |
| Butylene Glycol | 0 | 0 | 3 | 0 |
| Trisodium EDTA | 0,5 | 0,2 | 0,3 | 0,2 |
| Aqua | ad 100 | | | |
| Parfum | q.s. | q.s. | q.s. | q.s. |
| Resultat | Homogene Zubereitung | | | |

5. O/W Emulsionen - Die Beispiele 55 bis 57 sind nicht Teil der Erfindung

| INCI: O/W-Emulsion | 54 | 55 | 56 | 57 |
|---|---|---|---|---|
| Varisoft TA 100 (Distearyldimonium Chloride; Evonik Industries) | 1 | 1,25 | 1 | 1,25 |
| Glycerin | 25 | 30 | 28 | 27 |
| Lipocire NA-10/B Pastillen (Hydrogenated Coco-Glycerides; Gattefosse) | 2,5 | 2 | 2,5 | 2 |
| Stearyl Alcohol | 2 | 2 | 2 | 2 |
| Luviquat Excellence (Polyquaternium-16; BASF) | 2,5 | 0 | 0 | 0 |
| Luviquat FC 550 (Polyquaternium-16; BASF) | 0 | 2 | 0 | 0 |
| Luviquat FC 370 (Polyquaternium-16; BASF) | 0 | 0 | 1,75 | 0 |
| Luviquat Style (Polyquaternium-16; BASF) | 0 | 0 | 0 | 2,0 |
| Parfum | q.s. | q.s. | q.s. | q.s. |
| Octopirox (Piroctone Olamine; Clariant) | 0,05 | 0,05 | 0,05 | 0,05 |
| Aqua | Ad 100 | | | |
| Resultat: homogene Zubereitung | | | | |

**Patentansprüche**

1. Verwendung von einem oder mehreren Polyquaternium-16 Polymeren (3-Methyl-1-Vinylimidazolium Chlorid-1-Vinyl-2-Pyrrolidinon Chloride) als Deodorantwirkstoff **dadurch gekennzeichnet, dass** die Polyquaternium-16 Polymere

   • aus einem Monomerverhältnis von Vinylpyrrolidon zu quaternisiertem Vinylimidazol im Bereich von 4 bis 8 Gew.% zu 92 bis 96 Gew.% gebildet sind,
   • eine Ladungsdichte im Bereich von 4 bis 7 meq/g und
   • eine Molmasse im Bereich von 10.000 bis 1.000.000 aufweisen.

2. Verwendung von Polyquaternium-16 Polymeren nach Anspruch 1 in kosmetischen oder dermatologischen Zubereitungen.

3. Verwendung nach Anspruch 2 **dadurch gekennzeichnet, dass** die Polyquaternium Polymere in einer kosmetischen oder dermatologischen Zubereitung enthalten sind, die frei von organischen Verbindungen mit anionischer Ladung sind.

4. Verwendung nach einem der vorstehenden Ansprüche **dadurch gekennzeichnet, dass** die Polyquaternium Polymere in einer kosmetischen oder dermatologischen Zubereitung enthalten sind, die neben dem oder den Polyquaternium Polymeren frei von antitranspirant wirkenden Stoffen ist.

5. Verwendung nach einem der vorstehenden Ansprüche 2 bis 4 **dadurch gekennzeichnet, dass** der Anteil an einem oder mehreren Polyquaternium-16 Polymeren bis zu 10 Gew.%, insbesondere bis zu 2 Gew.%, bezogen auf die Gesamtmasse der Zubereitung gewählt wird.

6. Verwendung nach Anspruch 2 **dadurch gekennzeichnet, dass** die kosmetische oder dermatologische Zubereitung ein oder mehrere Stoffe gewählt aus den Gruppen b.) nichtionische Emulgatoren, c.) kationische Emulgatoren und d.) Rheologiemodifizierer umfasst, **dadurch gekennzeichnet, dass** der oder die Emulgatoren so zu wählen sind, dass der Gesamt HLB Wert der nicht ionischen Emulgatoren im Bereich von 3 bis 17 liegt.

7. Verwendung nach Anspruch 6 **dadurch gekennzeichnet, dass** die Zubereitung frei von organischen Verbindungen mit anionischer Ladung ist.

8. Verwendung nach Anspruch 6 oder 7 **dadurch gekennzeichnet, dass** die Polyquaternium-16 Polymere gewählt werden aus der Gruppe mit

   • einem Monomerverhältnis VP/QVI (Gew.%) im Bereich von 4 - 8/92 - 96, insbesondere 7/93 bis 5/95, insbesondere im Bereich von 5 bis 95% (VP/QVI),
   • einer Ladungsdichte (meq/g) im Bereich von 5 bis 6,5, bevorzugt zwischen 5,8 und 6,2 und
   • einer Molmasse (Da) im Bereich von 20.000 - 80.000.

9. Verwendung nach einem der vorstehenden Ansprüche 6 bis 8 **dadurch gekennzeichnet, dass** die Zubereitung

   b1.) ein oder mehrere nichtionische Emulgatoren mit einem HLB von größer 8 und
   b2.) ein oder mehrere nichtionischen Emulgator mit einem HLB kleiner 8 umfasst.

10. Verwendung nach einem der vorstehenden Ansprüche 6 bis 9 **dadurch gekennzeichnet, dass** als Emulgatoren b) ein oder mehrere Verbindungen aus der Gruppe Gruppe Glycol Distearate (HLB 1.0), Sorbitan Trioleate (HLB 1.8), Propylene Glycol Isostearate (HLB 2.5), Glycol Stearate (HLB 2.9), Glyceryl Isostearate (HLB 3,5), Sorbitan Sesquioleate (HLB 3.7), Glyceryl Stearate (HLB 3.8), Lecithin (HLB 4), Sorbitan Oleate (HLB 4.3), Sorbitan Monostearate NF (HLB 4.7), Sorbitan Stearate (HLB 4.7), Sorbitan Isostearate (HLB 4.7), Steareth-2 (HLB 4.9), Oleth-2 (HLB 4.9), Glyceryl Laurate (HLB 5.2), Ceteth-2 (HLB 5.3), PEG-30 Dipolyhydroxystearate (HLB 5.5), Glyceryl Stearate SE (HLB 5.8), Sorbitan Stearate (and) Sucrose Cocoate (HLB 6), PEG-4 Dilaurate (HLB 6), PEG-8 Dioleate (HLB 8), Sorbitan Laurate (HLB 8.6), PEG-40 Sorbitan Peroleate (HLB 9), Laureth-4 (HLB 9.7), PEG-7 Glyceryl Cocoate (HLB 10), PEG-20 Almond Glycerides (HLB 10), PEG-25 Hydrogenated Castor Oil (HLB 10.8), Stearamide MEA (HLB 11), Glyceryl Stearate (and) PEG-100 Stearate (HLB 11), Polysorbate 85 (HLB 11), PEG-7 Olivate (HLB 11), Cetearyl Glucoside (HLB 11), PEG-8 Oleate (HLB 11.6), Polyglyceryl-3 Methyglucose Distearate (HLB 12), Oleth-10 (HLB 12.4), Oleth-10 / Polyoxyl 10 Oleyl Ether NF (HLB 12.4), Ceteth-10 (HLB 12.9), PEG-8 Laurate (HLB 13), Ceteareth-12 (HLB 13,5), Cocamide MEA (HLB 13.5), PEG-30 Glyceryl Laurate (HLB 14), Polysorbate 60 NF (HLB 14.9), Polysorbate 60 (HLB 14.9), Polysorbate 80 (HLB 15), PEG-40 Hydrogenated Castor Oil (HLB 15), Isosteareth-20 (HLB 15), PEG-60 Almond Glycerides (HLB 15), Polysorbate 80 NF (HLB 15), PEG-20 Methyl Glucose Sesquistearate (HLB 15), Ceteareth-20 (HLB 15.2), Oleth-20 (HLB 15.3), Steareth-20 (HLB 15.3), Steareth-21 (HLB 15.5),Ceteth-20 (HLB 15.7), Isoceteth-20 (HLB 15.7), Polysorbate 20 (HLB 16.7), Polysorbate 20 NF (HLB 16.7), Laureth-23 (HLB 16.9), PEG-100 Stearate (HLB 18.8), Steareth-100 (HLB 18.8), PEG-80 Sorbitan Laurate (HLB 19.1), PEG-150Laurate(HLB 19.3) gewählt werden.

11. Verwendung nach Anspruch 9 oder 10 **dadurch gekennzeichnet, dass** als Emulgatoren b1.) mit einem HLB von größer 8 Ceteareth-20, Steareth-21, PEG-40 Hydrogenated Castor Oil, PG-10 Stearate, Isoceteth-20, Isosteareth-20 und/oder Ceteareth-12 und als Emulgatoren b2.) mit einem HLB von kleiner 8 Glyceryl Isostearate, Glyceryl Stearate und/oder Steareth-2 gewählt werden.

12. Verwendung nach einem der vorstehenden Ansprüche 6 bis 11 **dadurch gekennzeichnet, dass** die Zubereitungen auf einer Mikroemulsion, Makroemulsion, PIT-Emulsion oder alkoholischen Lösung basieren.

13. Verwendung nach Anspruch 12 dadurch gekennezichnet dass die Zubereitung eine alkoholische Lösung ist, welche **dadurch gekennzeichnet ist, dass** der Anteil an Alkohol, insbesondere Ethanol weniger als 50 Gew.%, bezogen auf die Gesamtmasse der Zubereitung, beträgt.

14. Verwendung nach einem der vorstehenden Ansprüche 6 bis 13 **dadurch gekennzeichnet, dass** die Zubereitungen auf einer Makroemulsion basiert und ein oder mehrere Triglyceride, bevorzugt mittelkettige Triglyceride, bevorzugt Caprylic/Capric Triglyceride, umfasst.

15. Verwendung nach Anspruch 12 oder 14 **dadurch gekennzeichnet, dass** die Zubereitungen auf einer Makroemulsion basiert und der Gesamt HLB-Wert im Bereich von 3 bis 14, vorzugsweise im Bereich 4 bis 12, idealerweise im Bereich 7 bis 10, liegt.

16. Verwendung nach einem der vorstehenden Ansprüche 6 bis 15 **dadurch gekennzeichnet, dass** der Anteil an einem oder mehreren Polyquaternium-16 Polymeren bis zu 10 Gew.%, insbesondere bis zu 2 Gew.%, bezogen auf die Gesamtmasse der Zubereitung gewählt wird.

## Claims

1. Use of one or more polyquaternium-16 polymers (3-methyl-1-vinylimidazolium chloride-1-vinyl-2-pyrrolidinone chloride) as deodorant active ingredient, **characterized in that** the polyquaternium-16 polymers

    • are formed from a monomer ratio of vinylpyrrolidone to quaternized vinylimidazole in the range of 4 to 8% by weight to 92 to 96% by weight
    • have a charge density in the range of 4 to 7 meq/g and
    • a molar mass in the range of 10 000 to 1 000 000.

2. Use of polyquaternium-16 polymers according to Claim 1 in cosmetic or dermatological preparations.

3. Use according to Claim 2, **characterized in that** the polyquaternium polymers are present in a cosmetic or dermatological preparation which is free of organic compounds having anionic charge.

4. Use according to any of the preceding claims, **characterized in that** the polyquaternium polymers are present in a cosmetic or dermatological preparation which, other than the polyquaternium polymer(s), is free of antiperspirant active substances.

5. Use according to any of preceding Claims 2 to 4, **characterized in that** the proportion of one or more polyquaternium-16 polymers is selected to an extent of up to 10% by weight, especially to an extent of up to 2% by weight, based on the total mass of the preparation.

6. Use according to Claim 2, **characterized in that** the cosmetic or dermatological preparation comprises one or more substances selected from the groups of b.) non-ionic emulsifiers, c.) cationic emulsifiers and d.) rheology modifiers, **characterized in that** the emulsifier(s) is/are to be selected such that the overall HLB value of the non-ionic emulsifiers is in the range of 3 to 17.

7. Use according to Claim 6, **characterized in that** the preparation is free of organic compounds having anionic charge.

8. Use according to Claim 6 or 7, **characterized in that** the polyquaternium-16 polymers are selected from the group having

    • a monomer ratio VP/QVI (% by weight) in the range of 4 - 8/92 - 96, especially 7/93 to 5/95, especially in the range of 5 to 95% (VP/QVI),
    • a charge density (meq/g) in the range of 5 to 6.5, preferably between 5.8 and 6.2 and
    • a molar mass (Da) in the range of 20 000 to 80 000.

9. Use according to any of preceding Claims 6 to 8, **characterized in that** the preparation comprises

b1.) one or more non-ionic emulsifiers having an HLB of greater than 8 and

b2.) one or more non-ionic emulsifiers having an HLB of less than 8.

**10.** Use according to any of preceding Claims 6 to 9, **characterized in that** the emulsifiers b) selected are one or more compounds from the group comprising glycol distearate (HLB 1.0), sorbitan trioleate (HLB 1.8), propylene glycol isostearate (HLB 2.5), glycol stearate (HLB 2.9), glyceryl isostearate (HLB 3.5), sorbitan sesquioleate (HLB 3.7), glyceryl stearate (HLB 3.8), lecithin (HLB 4), sorbitan oleate (HLB 4.3), sorbitan monostearate NF (HLB 4.7), sorbitan stearate (HLB 4.7), sorbitan isostearate (HLB 4.7), steareth-2 (HLB 4.9), oleth-2 (HLB 4.9), glyceryl laurate (HLB 5.2), ceteth-2 (HLB 5.3), PEG-30 dipolyhydroxystearate (HLB 5.5), glyceryl stearate SE (HLB 5.8), sorbitan stearate (and) sucrose cocoate (HLB 6), PEG-4 dilaurate (HLB 6), PEG-8 dioleate (HLB 8), sorbitan laurate (HLB 8.6), PEG-40 sorbitan peroleate (HLB 9), laureth-4 (HLB 9.7), PEG-7 glyceryl cocoate (HLB 10), PEG-20 almond glycerides (HLB 10), PEG-25 hydrogenated castor oil (HLB 10.8), stearamide MEA (HLB 11), glyceryl stearate (and) PEG-100 stearate (HLB 11), polysorbate 85 (HLB 11), PEG-7 olivate (HLB 11), cetearyl glucoside (HLB 11), PEG-8 oleate (HLB 11.6), polyglyceryl-3 methylglucose distearate (HLB 12), oleth-10 (HLB 12.4), oleth-10 / polyoxyl 10 oleyl ether NF (HLB 12.4), ceteth-10 (HLB 12.9), PEG-8 laurate (HLB 13), ceteareth-12 (HLB 13.5), cocamide MEA (HLB 13.5), PEG-30 glyceryl laurate (HLB 14), polysorbate 60 NF (HLB 14.9), polysorbate 60 (HLB 14.9), polysorbate 80 (HLB 15), PEG-40 hydrogenated castor oil (HLB 15), isosteareth-20 (HLB 15), PEG-60 almond glycerides (HLB 15), polysorbate 80 NF (HLB 15), PEG-20 methyl glucose sesquistearate (HLB 15), ceteareth-20 (HLB 15.2), oleth-20 (HLB 15.3), steareth-20 (HLB 15.3), steareth-21 (HLB 15.5), ceteth-20 (HLB 15.7), isoceteth-20 (HLB 15.7), polysorbate-20 (HLB 16.7), polysorbate 20 NF (HLB 16.7), laureth-23 (HLB 16.9), PEG-100 stearate (HLB 18.8), steareth-100 (HL 18.8), PEG-80 sorbitan laurate (HLB 19.1), PEG-150 laurate (HLB 19.3).

**11.** Use according to Claim 9 or 10, **characterized in that** the emulsifiers b1.) selected having an HLB of greater than 8 are ceteareth-20, steareth-21, PEG-40 hydrogenated castor oil, PEG-10 stearate, isoceteth-20, isosteareth-20 and/or ceteareth-12 and the emulsifiers b2.) selected having an HLB of less than 8 are glyceryl isostearate, glyceryl stearate and/or steareth-2.

**12.** Use according to any of preceding Claims 6 to 11, **characterized in that** the preparations are based on a microemulsion, macroemulsion, PIT emulsion or alcoholic solution.

**13.** Use according to Claim 12, **characterized in that** the preparation is an alcoholic solution which is **characterized in that** the proportion of alcohol, especially ethanol, is less than 50% by weight, based on the total mass of the preparation.

**14.** Use according to any of preceding Claims 6 to 13, **characterized in that** the preparations are based on a macroemulsion and comprise one or more triglycerides, preferably medium-chain triglycerides, preferably caprylic/capric triglycerides.

**15.** Use according to Claim 12 or 14, **characterized in that** the preparations are based on a macroemulsion and the overall HLB value is in the range of 3 to 14, preferably in the range 4 to 12, ideally in the range 7 to 10.

**16.** Use according to any of preceding Claims 6 to 15, **characterized in that** the proportion of one or more polyquaternium-16 polymers is selected to an extent of up to 10% by weight, especially to an extent of up to 2% by weight, based on the total mass of the preparation.

## Revendications

**1.** Utilisation d'un ou de plusieurs polymères de polyquaternium-16 (chlorure de 3-méthyl-1-vinylimidazolium-chlorure de 1-vinyl-2-pyrrolidinone) en tant qu'agent actif déodorant, **caractérisée en ce que** les polymères de polyquaternium-16

- sont formés à partir d'un rapport entre les monomères vinylpyrrolidone et vinylimidazole quaternisé dans la plage allant de 4 à 8 % en poids sur 92 à 96 % en poids,
- présentent une densité de charges dans la plage allant de 4 à 7 méq./g et
- une masse molaire dans la plage allant de 10 000 à 1 000 000.

**2.** Utilisation de polymères de polyquaternium-16 selon la revendication 1 dans des préparations cosmétiques ou dermatologiques.

**3.** Utilisation selon la revendication 2, **caractérisée en ce que** les polymères de polyquaternium sont contenus dans une préparation cosmétique ou dermatologique, qui est exempte de composés organiques contenant une charge anionique.

**4.** Utilisation selon l'une quelconque des revendications précédentes, **caractérisée en ce que** les polymères de polyquaternium sont contenus dans une préparation cosmétique ou dermatologique qui, outre le ou les polymères de polyquaternium, est exempte de substances à activité antitranspirante.

**5.** Utilisation selon l'une quelconque des revendications 2 à 4 précédentes, **caractérisée en ce que** la proportion du ou des polymères de polyquaternium-16 est de jusqu'à 10 % en poids, notamment de jusqu'à 2 % en poids, par rapport à la masse totale de la préparation.

**6.** Utilisation selon la revendication 2, **caractérisée en ce que** la préparation cosmétique ou dermatologique comprend une ou plusieurs substances choisies dans les groupes suivants : b) les émulsifiants non ioniques, c) les émulsifiants cationiques et d) les modificateurs de rhéologie, **caractérisée en ce que** le ou les émulsifiants sont choisis de telle sorte que la valeur HLB totale des émulsifiants non ioniques se situe dans la plage allant de 3 à 17.

**7.** Utilisation selon la revendication 6, **caractérisée en ce que** la préparation est exempt de composés organiques contenant une charge anionique.

**8.** Utilisation selon la revendication 6 ou 7, **caractérisée en ce que** les polymères de polyquaternium-16 sont choisis dans le groupe présentant :

- un rapport entre les monomères VP/QVI (% en poids) dans la plage allant de 4 à 8/92 à 96, notamment de 7/93 à 5/95, notamment dans la plage allant de 5 à 95 % (VP/QVI),
- une densité de charges (méq./g) dans la plage allant de 5 à 6,5, de préférence comprise entre 5,8 et 6,2, et
- une masse molaire (Da) dans la plage allant de 20 000 à 80 000.

**9.** Utilisation selon l'une quelconque des revendications 6 à 8 précédentes, **caractérisée en ce que** la préparation comprend :

b1) un ou plusieurs émulsifiants non ioniques ayant une valeur HLB supérieure à 8, et
b2) un ou plusieurs émulsifiants non ioniques ayant une valeur HLB inférieure à 8.

**10.** Utilisation selon l'une quelconque des revendications 6 à 9 précédentes, **caractérisée en ce qu'**un ou plusieurs composés du groupe constitué par Glycol Distearate (HLB 1,0), Sorbitan Trioleate (HLB 1,8), Propylene Glycol Isostearate (HLB 2,5), Glycol Stearate (HLB 2,9), Glyceryl Isostearate (HLB 3,5), Sorbitan Sesquioleate (HLB 3,7), Glyceryl Stearate (HLB 3,8), Lecithin (HLB 4), Sorbitan Oleate (HLB 4,3), Sorbitan Monostearate NF (HLB 4,7), Sorbitan Stearate (HLB 4,7), Sorbitan Isostearate, (HLB 4,7), Steareth-2 (HLB 4,9), Oleth-2 (HLB 4,9), Glyceryl Laurate (HLB 5,2), Ceteth-2 (HLB 5,3), PEG-30 Dipolyhydroxystearate (HLB 5,5), Glyceryl Stearate SE (HLB 5,8), Sorbitan Stearate (and) Sucrose Cocoate (HLB 6), PEG-4 Dilaurate (HLB 6), PEG-8 Dioleate (HLB 8), Sorbitan Laurate (HLB 8,6), PEG-40 Sorbitan Peroleate (HLB 9), Laureth-4 (HLB 9,7), PEG-7 Glyceryl Cocoate (HLB 10), PEG-20 Almond Glycerides (HLB 10), PEG-25 Hydrogenated Castor Oil (HLB 10,8), Stearamide MEA (HLB 11), Glyceryl Stearate (and) PEG-100 Stearate (HLB 11), Polysorbate 85 (HLB 11), PEG-7 Olivate (HLB 11), Cetearyl Glucoside (HLB 11), PEG-8 Oleate (HLB 11,6), Polyglyceryl-3 Methyglucose Distearate (HLB 12), Oleth-10 (HLB 12,4), Oleth-10/Polyoxyl 10 Oleyl Ether NF (HLB 12,4), Ceteth-10 (HLB 12,9), PEG-8 Laurate (HLB 13), Ceteareth-12 (HLB 13,5), Cocamide MEA (HLB 13,5), PEG-30 Glyceryl Laurate (HLB 14), Polysorbate 60 NF (HLB 14,9), Polysorbate 60 (HLB 14,9), Polysorbate 80 (HLB 15), PEG-40 Hydrogenated Castor Oil (HLB 15), Isosteareth-20 (HLB 15), PEG-60 Almond Glycerides (HLB 15), Polysorbate 80 NF (HLB 15), PEG-20 Methyl Glucose Sesquistearate (HLB 15), Ceteareth-20 (HLB 15,2), Oleth-20 (HLB 15,3), Steareth-20 (HLB 15,3), Steareth-21 (HLB 15,5), Ceteth-20 (HLB 15,7), Isoceteth-20 (HLB 15,7), Polysorbate 20 (HLB 16,7), Polysorbate 20 NF (HLB 13,5), Laureth-23 (HLB 16,9), PEG-100 Stearate (HLB 18,8), Steareth-100 (HLB 18,8), PEG-80 Sorbitan Laurate (HLB 19,1), PEG-150 Laurate(HLB 19,3) sont choisis en tant qu'émulsifiants b) .

**11.** Utilisation selon la revendication 9 ou 10, **caractérisée en ce que** Ceteareth-20, Steareth-21, PEG-40 Hydrogenated Castor Oil, PEG-10 Stearate, Isoceteth-20, Isosteareth-20 et/ou Ceteareth-12 sont choisis en tant qu'émulsifiants b1) ayant une valeur HLB supérieure à 8, et Glyceryl Isostearate, Glyceryl Stearate et/ou Steareth-2 sont choisis en tant qu'émulsifiants b2) ayant une valeur HLB inférieure à 8.

**12.** Utilisation selon l'une quelconque des revendications 6 à 11 précédentes, **caractérisée en ce que** les préparations sont à base d'une microémulsion, d'une macroémulsion, d'une émulsion PIT ou d'une solution alcoolique.

**13.** Utilisation selon la revendication 12, **caractérisée en ce que** la préparation est une solution alcoolique, qui est **caractérisée en ce que** la proportion d'alcool, notamment d'éthanol, est inférieure à 50 % en poids, par rapport à la masse totale de la préparation.

**14.** Utilisation selon l'une quelconque des revendications 6 à 13 précédentes, **caractérisée en ce que** les préparations sont à base d'une macroémulsion, et comprennent un ou plusieurs triglycérides, de préférence des triglycérides à chaîne moyenne, de préférence le triglycérique caprylique/caprique.

**15.** Utilisation selon la revendication 12 ou 14, **caractérisée en ce que** les préparations sont à base d'une macroémulsion, et la valeur HLB totale se situe dans la plage allant de 3 à 14, de préférence dans la plage allant de 4 à 12, idéalement dans la plage allant de 7 à 10.

**16.** Utilisation selon l'une quelconque des revendications 6 à 15 précédentes, **caractérisée en ce que** la proportion d'un ou de plusieurs polymères de polyquaternium-16 est choisie jusqu'à 10 % en poids, notamment jusqu'à 2 % en poids, par rapport à la masse totale de la préparation.

Abbildung 1

Suspensionstest
*S. epidermidis*
*20 min Inkubation*

EP 3 171 943 B1

Abbildung 2

Abbildung 3

**Wirksamkeit im Stoff**

*S.epidermidis*

N= 4

EP 3 171 943 B1

**Abbildung 4**

EP 3 171 943 B1

Abbildung 5

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2013052454 A1
- EP 1504744 B1
- US 20070184016 A1
- WO 2007095008 A2
- EP 1991654 A1
- DE 102010007958 A1
- JP 2014101356 A
- WO 9401077 A1

- EP 200548 A2
- WO 199524105 A1
- US 4743440 A
- WO 2003030853 A2
- WO 2009091794 A1
- EP 1761241 A1
- JP 2014101356 B
- WO 1998015254 A1

**In der Beschreibung aufgeführte Nicht-Patentliteratur**

- Fett-, Öl-, Kosmetik-, Pharma- und Waschmittelindustrie. Marz 1985

- *J. Soc. Cosmet. Chem.,* Juli 1987, vol. 38, 223-231